# EUROPEAN PATENT SPECIFICATION

(11) **EP 3 441 052 B1**
(45) Date of publication and mention of the grant of the patent: **08.07.2020**
(21) Application number: 16897945.8
(22) Date of filing: 08.04.2016
(51) Int. Cl.: A61F 13/15, B65H 21/02, B65H 23/025, B65H 23/188, B65H 39/14

(54) **MANUFACTURING METHOD AND MANUFACTURING DEVICE FOR SHEET-LIKE MEMBER FOR ABSORBENT ARTICLE**
HERSTELLUNGSVERFAHREN UND HERSTELLUNGSVORRICHTUNG FÜR BLATTFÖRMIGES ELEMENT FÜR SAUGFÄHIGEN ARTIKEL
PROCÉDÉ DE FABRICATION ET DISPOSITIF DE FABRICATION POUR ÉLÉMENT DE TYPE FEUILLE POUR ARTICLE ABSORBANT

(43) Date of publication of application: 13.02.2019
(73) Proprietor: Unicharm Corporation, Shikokuchuo-shi, Ehime 799-0111 (JP)
(72) Inventor: HAGITA, Hiromi, Kanonji-shi Kagawa 769-1602 (JP); SAKATA, Rei, Kanonji-shi Kagawa 769-1602 (JP)
(74) Representative: Staeger & Sperling Partnerschaftsgesellschaft mbB
(86) International application number: PCT/JP2016/061568
(87) International publication number: WO 2017/175389

(56) References cited:
- EP-A1- 1 983 395
- WO-A1-98/21134
- JP-A- H04 314 443
- JP-A- 2001 517 098
- JP-B1- 5 895 107
- JP-B2- 3 373 611
- JP-B2- 5 707 467
- US-A1- 2012 090 071
- US-A1- 2015 250 655

## Description

### [Technical Field]

The invention relates to a method and an apparatus for manufacturing a sheet-like member associated with an absorbent article such as a disposable diaper.

### [Background Art]

In a manufacturing line of a disposable diaper, which is an example of a conventional absorbent article, the direction of transport of a continuous sheet is set to a direction in which the continuous sheet continues, and the continuous sheet is sent and supplied to a processing line, which is an example of a processing section. In the processing line, an appropriate processing device performs a processing at direction-of-transport intervals, and a portion of the continuous sheet which is to be a diaper undergoes the processing, producing a diaper. In order to process a portion which is to be a diaper, the processing device usually processes the continuous sheet based on a synchronization signal which determines by a product pitch in the direction of transport.

### [Citation List]

### [Patent Literature]

[PTL 1] Japanese Unexamined Patent Application Publication (Translation of PCT Application) No. 2014-507233
[PTL 2] US Patent Application Publication No. 2004-0030432.

Further prior art in this technical field is disclosed in documents WO 98/21134, US 2012/090071 A1 and EP 1 983 395 A1.

### [Summary of Invention]

### [Technical Problem]

The abovementioned continuous sheet is made of nonwoven fabric or film. In some cases, nonuniformity of material (e.g., basis weight) can exist at a large pitch in a direction in which the continuous sheet continues. For example, in some cases, material of the continuous sheet varies in a long cycle which corresponds to the direction-of-continuing length of tens to hundreds of diapers. In this case, on the continuous sheet, easy-to-stretch portions and hard-to-stretch portions exist alternately in the abovementioned long cycle.

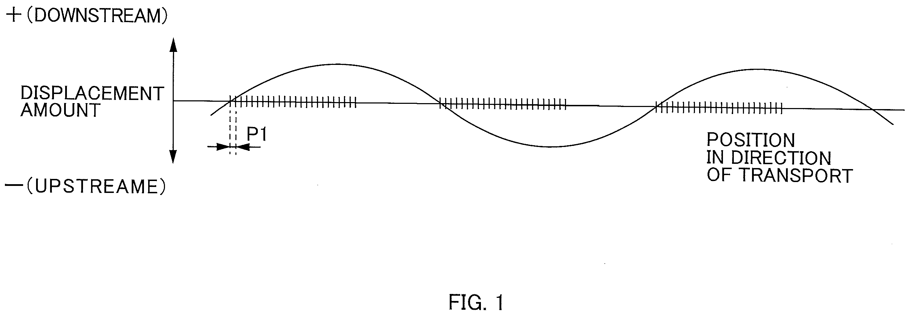

Accordingly, even if such a continuous sheet is transferred to the processing line at a constant tension value (N), the amount of stretching of the continuous sheet in the processing line can vary at the abovementioned large pitch. FIG. 1 is a graph explaining it; the horizontal axis shows the position in the direction of transport, and the vertical axis shows the amount of the direction-of-transport displacement of the position of the continuous sheet from the proper position. Note that, the plus side means shift to downstream, and the minus side means shift to upstream. In the horizontal axis, also indicated is the abovementioned product pitch P1 corresponding to one diaper.

As is apparent from FIG. 1, in the continuous sheet, there are repeatedly emerged a portion located downstream with respect to the correct proper position and a portion located upstream with respect to the same, in a long cycle corresponding to tens to hundreds of diapers.

In the case where the amount of stretching of a continuous sheet varies, even if the processing device processes the continuous sheet at a product pitch P1 based on the abovementioned synchronization signal, it is impossible to perform the processing at a proper position on the continuous sheet.

With regards to this, the following configuration is conceivable as a reference example: a trace of the processing left on the continuous sheet is detected, and based on the detection result, the processing device corrects or adjusts the timing of processing, and processes the continuous sheet.

However, even in the case of reference example, it is not suppress variation of the amount of stretching of the continuous sheet, which is the root cause. Accordingly, it is substantially difficult to perform the processing at the proper position in the continuous sheet, making it difficult to produce a proper diaper.

The invention has been made in view of the above problems, and an advantage thereof is to make it possible to perform a processing at a proper position on a continuous sheet.

### [Solution to Problem]

The invention to achieve the above advantage is a method according to claim 1 for manufacturing a sheet-like member using a continuous sheet,
the sheet-like member associated with an absorbent article,
the method including:
   a supplying step of supplying the continuous sheet,
      the supplying step being performed by transferring the continuous sheet to a processing section by transporting the continuous sheet in a direction of transport,
      the direction of transport being a direction in which the continuous sheet continues;
   a processing step of processing a portion of the continuous sheet by the processing section at an interval in the direction of transport,
      the portion being to be the absorbent article,
      the processing step being performed at a predetermined position in the direction of transport;
   a detecting step of detecting a trace of the processing left on the continuous sheet and outputting a detection result,
      the supplying step including adjusting a direction-of-transport tension value of the continuous sheet based on the detection result,
      the direction-of-transport tension value of the continuous sheet being a tension value at a time of transferring the continuous sheet to the processing section.

Further, an apparatus for manufacturing a sheet-like member using a continuous sheet according to the invention is defined in claim 10,
the sheet-like member associated with an absorbent article,
the apparatus including:
   a supplying section that supplies the continuous sheet by transferring the continuous sheet to a processing section by transporting the continuous sheet in a direction of transport,
      the direction of transport being a direction in which the continuous sheet continues;
   a processing device that processes a portion of the continuous sheet by the processing section at an interval in the direction of transport,
      the portion being to be the absorbent article; and
   a detecting device that detects a trace of the processing left on the continuous sheet and that outputs a detection result,
the supplying section configured to adjust a direction-of-transport tension value of the continuous sheet based on the detection result,
   the direction-of-transport tension value of the continuous sheet being a tension value at the time of transferring the continuous sheet to the processing section.

Other features of this invention will become apparent from the description in this specification and the attached drawings.

### [Advantageous Effects of Invention]

According to the invention, it is possible to perform a processing at a proper position on a continuous sheet.

### [Brief Description of Drawings]

[FIG. 1] FIG. 1 is a graph showing that the amount of stretching of a continuous sheet, which is a material of a diaper 1, varies at a large pitch.
[FIG. 2] FIG. 2A is a schematic plan view of an example of a disposable diaper 1 in an unfolded state, and FIG. 2B is a cross-sectional view taken along line B-B in FIG. 2A.
[FIG. 3] FIG. 3 is a schematic plan view showing how a diaper 1 is produced.
[FIG. 4] FIG. 4 is a schematic side view of a manufacturing line IM of a diaper 1 according to the present embodiment.
[FIG. 5] FIG. 5 is a schematic side view of a back-sheet processing system L4a.
[FIG. 6] FIG. 6 is a schematic side view of a top-sheet processing system L3a.
[FIG. 7] FIG. 7 is a schematic side view showing an example in which an imaging position P38 for an imaging device 38C is located downstream in the direction of transport with respect to a merging position Pj.
[FIG. 8] FIG. 8A is a schematic plan view of a back-sheet processing system L4a according to the present embodiment, and FIG. 8B is a schematic plan view of a back-sheet processing system L4a according to a modified example.
[FIG. 9A] FIG. 9A is a schematic plan view showing an example in which two tension adjustment devices 43A and 43B are provided.
[FIG. 9B] FIG. 9B is a schematic side view in which a view along arrows C-C in FIG. 9A and a view along arrows D-D in FIG. 9A are connected and illustrated in a plane.
[FIG. 10] FIG. 10 is a schematic side view of an example of a tension adjustment device 44 which is not a dancer-roll type.

### [Description of Embodiments]

At least the following matters will be made clear by the description in the present specification and the accompanying drawings.

A method for manufacturing a sheet-like member using a continuous sheet,
the sheet-like member associated with an absorbent article,
the method including:
   a supplying step of supplying the continuous sheet,
      the supplying step being performed by transferring the continuous sheet to a processing section by transporting the continuous sheet in a direction of transport,
      the direction of transport being a direction in which the continuous sheet continues;
   a processing step of processing a portion of the continuous sheet by the processing section at an interval in the direction of transport,
      the portion being to be the absorbent article,
      the processing step being performed at a predetermined position in the direction of transport;
   a detecting step of detecting a trace of the processing left on the continuous sheet and outputting a detection result,
      the supplying step including adjusting a direction-of-transport tension value of the continuous sheet based on the detection result,
      the direction-of-transport tension value of the continuous sheet being a tension value at a time of transferring the continuous sheet to the processing section.

With such a method for manufacturing a sheet-like member associated with an absorbent article, the trace of the processing left on the continuous sheet is detected, and based on the detection result, the tension value at the time of transferring the continuous sheet to the processing section is adjusted. Accordingly, in the case where the continuous sheet is transferred with the abovementioned tension value being constant, though nonuniformity of material of the continuous sheet itself could cause variation of the amount of the direction-of-transport stretching of the continuous sheet, that variation can be suppressed, making it possible to perform the processing at a proper position on the continuous sheet.

In such a method for manufacturing a sheet-like member associated with an absorbent article, it is according to the invention
that, if the detection result indicates that the trace of the processing is located downstream in the direction of transport with respect to a target position in the continuous sheet,
   the tension value is increased, and
that, if the detection result indicates that the trace of the processing is located upstream in the direction of transport with respect to the target position,
   the tension value is decreased.

With such a method for manufacturing a sheet-like member associated with an absorbent article, in the case where the detection result indicates that the trace of the processing is located downstream with respect to the target position, the tension value is increased. In the case where the detection result indicates that the trace of the processing is located upstream with respect to the target position, the tension value is decreased. This makes it possible to suppress variation of the amount of the direction-of-transport stretching of the continuous sheet in the processing device. This makes it possible for the processing device to perform the processing at a proper position on the continuous sheet.

In such a method for manufacturing a sheet-like member associated with an absorbent article, it is desirable
that, in the processing section, the continuous sheet is transported in a predetermined transport path, and
that a direction-of-transport length of the transport path keeps constant.

With such a method for manufacturing a sheet-like member associated with an absorbent article, the abovementioned transport-path length in the processing section keeps constant. This makes it possible to prevent large shift of the timing of the processing from the timing corresponding to the proper position in the continuous sheet.

In such a method for manufacturing a sheet-like member associated with an absorbent article, it is according to the invention
that the processing is defined as an upstream processing,
that another processing is defined as a downstream processing,
that the downstream processing is performed, at an interval in the direction of transport, at a position downstream in the direction of transport with respect to a position where the upstream processing is performed,
that a portion of the continuous sheet that is to be the absorbent article is subject to the downstream processing, and
that the trace of the processing is detected at a position downstream with respect to the position where the downstream processing is performed.

With such a method for manufacturing a sheet-like member associated with an absorbent article, the amount of displacement of the trace of the upstream processing from the proper position can be determined with high accuracy. Variation of the amount of stretching of the continuous sheet accumulates at a position downstream in the direction of transport. Accordingly, the amount of displacement of the trace of the abovementioned upstream processing from the proper position in the continuous sheet tends to be more apparent at a position downstream with respect to the position where the downstream processing is performed, compared to a position between the position where the upstream processing is performed and the position where the downstream processing is performed. Consequently, with the abovementioned manufacturing method, the amount of displacement of the trace of the upstream processing from the proper position can be determined with high accuracy.

In such a method for manufacturing a sheet-like member associated with an absorbent article, it is desirable
that, in the supplying step, a feeding device that feeds the continuous sheet from a material coil is used,
that, in the feeding device, the continuous sheet is fed and transported while a direction of feeding of the continuous sheet being X-direction as viewed from above,
that, in the processing section, the continuous sheet is transported while the direction of transport of the continuous sheet being Y-direction as viewed from above, the Y-direction intersecting the X-direction,
that a bar-shaped direction-of-transport changing member is arranged at a position in the direction of transport between the feeding device and the processing section,
that the direction of transport of the continuous sheet changes from the X-direction to the Y-direction by wrapping the continuous sheet around the direction-of-transport changing member, and
that the tension adjustment device is arranged at a position in the direction of transport between the direction-of-transport changing member and the processing section.

With such a method for manufacturing a sheet-like member associated with an absorbent article, the tension adjustment device is arranged close to the processing section. This makes it possible to transfer to the processing section the continuous sheet whose tension value is adjusted.

There is a risk that variation of the amount of stretching of the continuous sheet is promoted due to sliding between the direction-of-transport changing member and the continuous sheet. But, with regards to this, in this method, the tension value at the time of transferring the continuous sheet to the processing section is adjusted based on the abovementioned detection result, suppressing variation of the amount of stretching of the continuous sheet as mentioned above. This makes it possible to effectively achieve the abovementioned effect that a processing is performed at a proper position on the continuous sheet.

In such a method for manufacturing a sheet-like member associated with an absorbent article, it is desirable
that the tension adjustment device is defined as a first tension adjustment device,
that a second tension adjustment device is arranged between the feeding device and the direction-of-transport changing member in the direction of transport, and
that the second tension adjustment device is for controlling a feeding operation of the feeding device so that a tension value of the continuous sheet that has been fed from the feeding device becomes a predetermined value.

With such a method for manufacturing a sheet-like member associated with an absorbent article, the continuous sheet passes the direction-of-transport changing member while the tension value of the continuous sheet being adjusted to the predetermined value by the second tension adjustment device. Accordingly, it can be dealt without serious problems even if the optimal tension value at the time of passing the direction-of-transport changing member is different from the optimal tension value at the time of transferring the continuous sheet to the processing section.

In such a method for manufacturing a sheet-like member associated with an absorbent article, it is desirable that
in the processing section, a transport speed at which the continuous sheet is transported in the direction of transport is changed.

With such a method for manufacturing a sheet-like member associated with an absorbent article, there is a risk that a disturbance, which is change of the transport speed in the processing section, promotes variation of the amount of stretching of the continuous sheet. But, with regards to this, in this method, the tension value at the time of transferring the continuous sheet to the processing section is adjusted based on the abovementioned detection result, suppressing variation of the amount of stretching of the continuous sheet, as mentioned above. This makes it possible to effectively achieve the abovementioned effect that a processing is performed at a proper position on the continuous sheet.

In such a method for manufacturing a sheet-like member associated with an absorbent article, it is desirable that
a timing at which the processing is performed in the processing step is shifted based on the detection result.

With such a method for manufacturing a sheet-like member associated with an absorbent article, a timing at which the abovementioned processing is performed is shifted based on the abovementioned detection result. This makes it possible to perform the processing at a proper position in the continuous sheet.

In such a method for manufacturing a sheet-like member associated with an absorbent article, it is desirable that
that a first continuous sheet is the continuous sheet,
that a second continuous sheet is a continuous sheet different from the first continuous sheet, and
that, at a merging position downstream in the direction of transport with respect to a position where the processing is performed,
the second continuous sheet is transferred and bonded with the first continuous sheet.

With such a method for manufacturing a sheet-like member associated with an absorbent article, it is possible to manufacture the sheet-like member including the first continuous sheet and the second continuous sheet.

In such a method for manufacturing a sheet-like member associated with an absorbent article, it is desirable
that the method further comprises:
a second supplying step of supplying the second continuous sheet,
   the second supplying step being performed by transferring the second continuous sheet to a second processing section by transporting the second continuous sheet in a direction of transport,
   the direction of transport being a direction in which the second continuous sheet continues;
a second processing step of performing a second processing to a portion of the second continuous sheet by the second processing section at an interval in the direction of transport,
   the portion being to be the absorbent article,
   the second processing step being performed at a predetermined position in the direction of transport; and
a second detecting step of detecting a trace of the second processing left on the second continuous sheet and outputting a second detection result,
that the second supplying step includes adjusting a direction-of-transport tension value of the continuous sheet based on the second detection result,
that the direction-of-transport tension value of the continuous sheet being a tension value at the time of transferring the continuous sheet to the second processing section, and
that the merging position is located downstream in the direction of transport with respect to a position where the second processing is performed.

With such a method for manufacturing a sheet-like member associated with an absorbent article, the first continuous sheet and the second continuous sheet are bonded while suppressing variation of the amount of stretching of the sheets. This makes it possible to prevent a shape-related or size-related trouble of the absorbent article such as size irregularity, which could be caused in the case where these first and second continuous sheets are bonded under the condition that the variation is not suppressed.

In such a method for manufacturing a sheet-like member associated with an absorbent article, it is desirable that
a trace of the processing left on the first continuous sheet is detected at a position downstream in the direction of transport with respect to the merging position.

With such a method for manufacturing a sheet-like member associated with an absorbent article, the trace of the processing left on the first continuous sheet is detected at the position downstream with respect to the merging position. This can ensure a large direction-of-transport distance from the position where the processing is performed to the detecting position. This makes it possible to detect the trace of the processing while the amount of the displacement of the trace of the processing from the proper position in the first continuous sheet tends to be apparent. Consequently, the amount of displacement of the trace of the processing from the proper position can be determined with high accuracy.

If necessary, an identical imaging device can detect both of the trace of the processing associated with the first continuous sheet and the trace of the second processing associated with the second continuous sheet. This enables to simplify the configuration of devices.

Further,
An apparatus for manufacturing a sheet-like member using a continuous sheet,
the sheet-like member associated with an absorbent article,
the apparatus including:
a supplying section that supplies the continuous sheet by transferring the continuous sheet to a processing section by transporting the continuous sheet in a direction of transport,
   the direction of transport being a direction in which the continuous sheet continues;
      a processing device that processes a portion of the continuous sheet by the processing section at an interval in the direction of transport,
   the portion being to be the absorbent article; and
a detecting device that detects a trace of the processing left on the continuous sheet and that outputs a detection result,
the supplying section configured to adjust a direction-of-transport tension value of the continuous sheet based on the detection result,
   the direction-of-transport tension value of the continuous sheet being a tension value at the time of transferring the continuous sheet to the processing section.

With such an apparatus for manufacturing a sheet-like member associated with absorbent article, it is possible to achieve the same effects as those of the foregoing manufacturing method.

### === The Present Embodiment ===

A method and an apparatus for manufacturing a sheet-like member associated with an absorbent article according to the present embodiment are used in the manufacturing line LM of a disposable diaper 1. That is, a method and an apparatus of the present embodiment are applied to manufacturing of a disposable diaper 1, which is an example of an absorbent article.

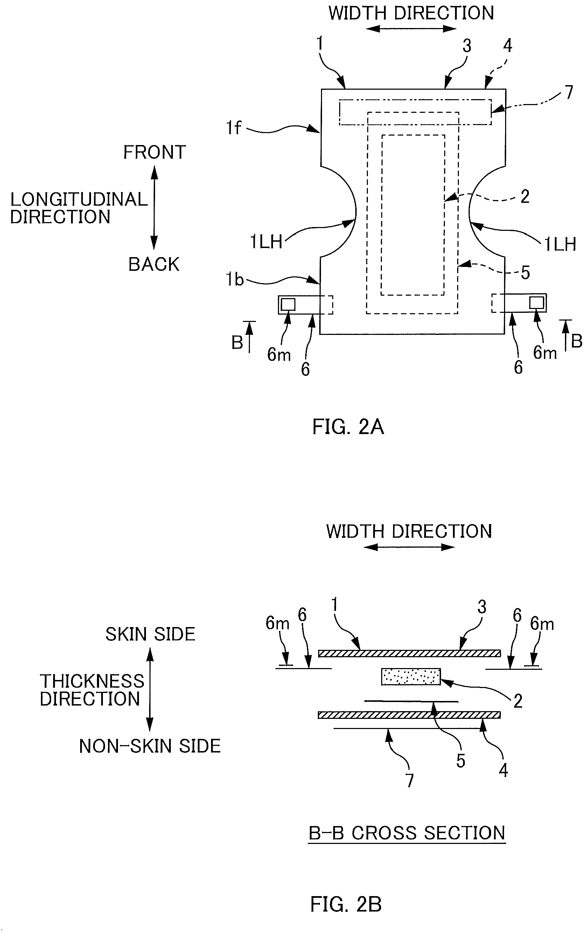

FIG. 2A is a schematic plan view of an example of the disposable diaper 1 in an unfolded state. FIG. 2B is a cross-sectional view taken along line B-B in FIG. 2A.

The diaper 1 is a so-called tape-type diaper 1. That is, it is a diaper which is put on a wearer using a fastening-tape member 6 and a target tape 7. In an unfolded state shown in FIG. 2A in which the diaper 1 is opened by disengaging the fastening-tape member 6 and the target tape 7, the diaper 1 has three directions perpendicular to one another: a longitudinal direction, a width direction and a thickness direction.

The diaper 1 includes: an absorbent body 2 that absorbs excrement such as urine; a liquid-permeable top sheet 3 provided on the skin side of the absorbent body 2; a back sheet 4 provided on the non-skin side of the absorbent body 2; and a liquid-impermeable leak-proof sheet 5 provided between the absorbent body 2 and the back sheet 4, preventing excrement from leaking to the non-skin side.

In the unfolded state shown in FIG. 2A, the top sheet 3 and the back sheet 4 have an identical, substantially hourglass shape as viewed from above. Accordingly, of the substantially hourglass shape, a portion narrowing inwardly in the width direction serve as leg openings 1LH and 1LH when it is put on.

When the diaper is put on, the longitudinal one-side part 1f and the longitudinal other-side part 1b of the unfolded diaper 1 respectively serve as a front part 1f covering wearer's front side and a back part 1b covering wearer's back side. Accordingly, in order to enable to keep the shape of the diaper 1 when being put on, the back part 1b includes a pair of fastening-tape members 6 and 6 each having a male member 6m of a hook-and-loop fastener and respectively projecting toward the sides in the width direction. The front part 1f has a target tape 7 on its non-skin-side surface, which is made of nonwoven fabric or a female member of a hook-and-loop fastener for fastening the abovementioned male member 6m when the diaper is put on.

In this example, the absorbent body 2 includes: an absorbent core made of liquid absorbent fiber such as pulp fiber and having a substantially rectangular shape as viewed from above; and a liquid-permeable wrapping sheet that is made of tissue paper or nonwoven fabric and that wraps the outer circumferential surface of the absorbent core. However, the invention is not limited thereto.

The materials of the top sheet 3 and the back sheet 4 can be exemplified by nonwoven fabric containing thermoplastic resin fiber made of substance such as polyethylene and polypropylene, and the material of the leak-proof sheet 5 can be exemplified by thermoplastic resin film made of substance such as polyethylene. However, the invention is not limited thereto. For example, as for materials of the top sheet 3 and the back sheet 4, a material having an appropriate stretchability in the longitudinal direction may be employed.

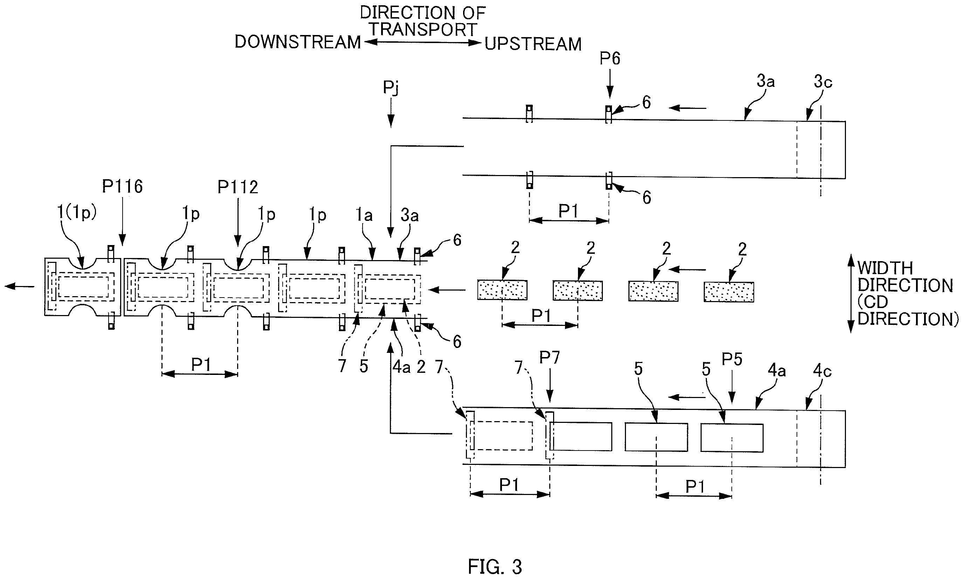

FIG. 3 is a schematic plan view showing how the diaper 1 is produced.

The top sheet 3 and the back sheet 4 are brought into the manufacturing line LM in the form of respective material coils 3C and 4C in which continuous sheets 3a and 4a (the materials) are wound in a coil-like manner.

First, the continuous sheet 4a of the back sheet which has been fed from a material coil 4C is transported in the direction of transport, which is a direction in which the continuous sheet 4a continues. During the transportation, at a predetermined position P5 in the direction of transport, a single sheet of the leak-proof sheet 5 is bonded onto the skin-side surface of the continuous sheet 4a at intervals of a product pitch P1 in the direction of transport. At a predetermined position P7 downstream therefrom, a single piece of the target tape 7 is bonded onto the non-skin-side surface of the continuous sheet 4a at intervals of the product pitch P1 in the direction of transport. Then, the continuous sheet 4a is transported to the merging position Pj where it merges with the continuous sheet 3a of the top sheet.

The continuous sheet 3a of the top sheet is fed from another material coil 3C, and is transported in the direction of transport, which is a direction in which the continuous sheet 3a continues. During the transportation, the at a predetermined position P6 in the direction of transport, a pair of fastening-tape members 6 and 6 arranged in the width direction are bonded onto the non-skin-side surface of the continuous sheet 3a at intervals of the product pitch P1 in the direction of transport. Then, the continuous sheet 3a is transported to the abovementioned merging position Pj.

At the merging position Pj, the absorbent body 2 also merges. Specifically, a plurality of the absorbent bodies 2, 2,... are transported toward the merging position Pj while being aligned at the product pitch P1 in the direction of transport. At the merging position Pj, the continuous sheet 4a of the back sheet merges with the absorbent body 2 from the non-skin side of the absorbent body 2. And, the continuous sheet 3a of the top sheet merges with the absorbent body 2 from the skin side of the absorbent body 2. Accordingly, these three components are bonded as a single unit with adhesive, producing the substrate 1a of the diaper. Specifically, in the substrate 1a of the diaper, parts 1p each of which is finally to be a diaper 1 are aligned continuously at the product pitch P1 in the direction of transport.

The substrate 1a of the diaper is subsequently transported in the direction of transport. At a predetermined position P112 in the direction of transport, cut-outs are made in the widthwise ends of the substrate 1a, at the product pitch P1 in the direction of transport. Accordingly, the parts 1p each of which is finally to be a diaper each becomes in the abovementioned substantially hourglass shape as viewed from above. At a position P116 downstream therefrom, the substrate 1a is cut by the product pitch P1 in the direction of transport. Accordingly, the most downstream part 1p of the substrate 1a is cut and separated, producing a diaper 1.

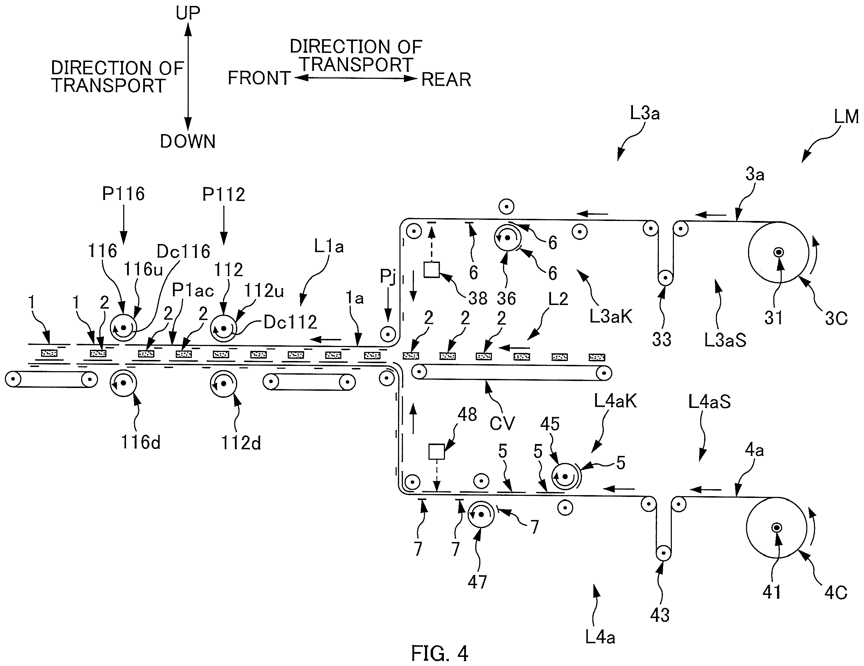

FIG. 4 is a schematic side view of the manufacturing line LM of the diaper 1.

The manufacturing line LM includes: a back-sheet processing system L4a which processes the continuous sheet 4a of the back sheet; a top-sheet processing system L3a which processes the continuous sheet 3a of the top sheet; an absorbent-body producing system L2 which produces the absorbent body 2; and a substrate processing system L1a which processes the substrate 1a of a diaper. The back-sheet processing system L4a, the top-sheet processing system L3a and the absorbent-body producing system L2 merge at the abovementioned merging position Pj. Downstream from the merging position Pj, the abovementioned substrate processing system L1a is connected.

In the substrate processing system L1a, processing the substrate 1a of the diaper is sometimes substantially equivalent to processing the continuous sheet 4a of the back sheet or processing the continuous sheet 3a of the top sheet. Accordingly, it can be said that the back-sheet processing system L4a and the top-sheet processing system L3a continuously exist in the substrate processing system L1a, not end at the merging position Pj. Therefore, in a narrower sense, the back-sheet processing system L4a and the top-sheet processing system L3a both end at the merging position Pj. And, in a wider sense, the back-sheet processing system L4a and the top-sheet processing system L3a both exist extending in the substrate processing system L1a, not end at the merging position Pj.

In the systems L4a, L3a, L2 and L1a, there are arranged appropriate transport devices such as belt conveyers and transport rollers. Accordingly, unless otherwise stated, these transport devices transports to-be-transported things (e.g., the continuous sheets 4a and 3a) in the direction of transport. It should be noted that, a belt conveyer is exemplified by an ordinary belt conveyor having an endless belt which is driven and rotated and which serves as a transport surface, and is exemplified by a suction belt conveyor having an endless belt whose outer circumferential surface has absorption function.

In the manufacturing line LM, the width direction of the manufacturing line LM is defined as a CD direction (a direction penetrating the paper plane of FIG. 4). In this example, CD direction is oriented in the horizontal direction. However, the invention is not limited thereto. And, in this example, two directions perpendicular to CD direction are defined as a vertical, up-down direction and a horizontal, front-rear direction. Accordingly, each of the directions in which the continuous sheets 4a and 3a for the back sheet and the top sheet are transported is oriented in a direction specified by the up-down direction and the front-rear direction, depending on a position in the direction of transport. The width directions of the continuous sheets 4a and 3a for the back sheet and the top sheet are each parallel to CD direction. Directions perpendicular to CD direction and the direction of transport are defined as Z-direction, and the Z-direction is parallel to the thickness directions of the continuous sheets 4a and 3a for the back sheet and the top sheet.

### <<< Back-Sheet Processing System L4a>>>

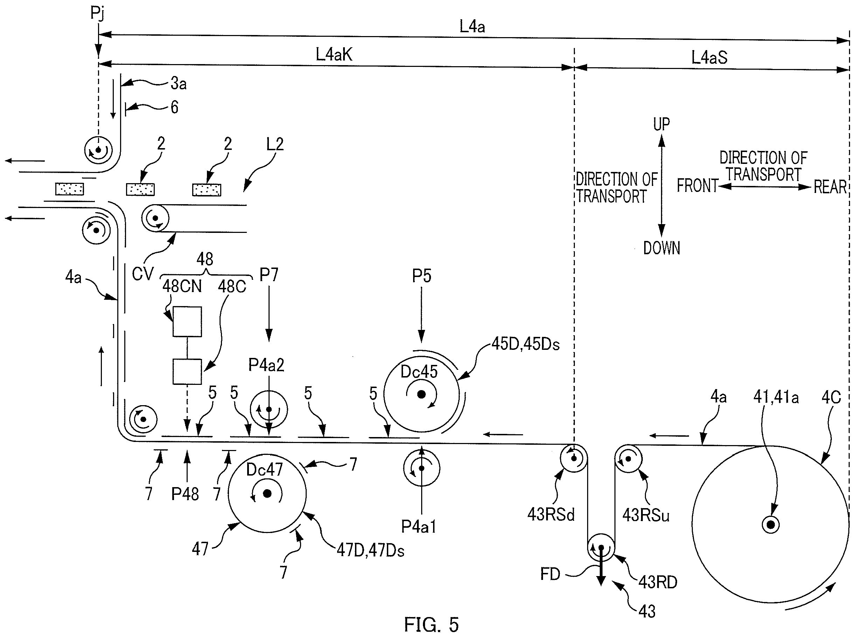

FIG. 5 is a schematic side view of the back-sheet processing system L4a.

The back-sheet processing system L4a includes: a back-sheet supply line L4aS (corresponding to the supplying section); and a back-sheet processing line L4aK (corresponding to the processing section) arranged downstream from the supply line L4aS in the direction of transport. In the back-sheet supply line L4aS, the continuous sheet 4a of the back sheet (corresponding to the continuous sheet or the first continuous sheet) is fed and supplied from the material coil 4C (corresponding to the supplying step) . In the back-sheet processing line L4aK, a certain process is performed to the continuous sheet 4a of the back sheet which is supplied from the back-sheet supply line L4aS and transported in the direction of transport (corresponding to the processing step).

### (1) Back-Sheet Supply Line L4aS

The back-sheet supply line L4aS includes: a feeding device 41 for feeding the continuous sheet 4a of the back sheet from the material coil 4C; and a tension adjustment device 43 which adjusts to a target value the direction-of-transport tension value (N) of the continuous sheet 4a which is fed by the device 41 and is being transported.

As shown in FIG. 5, the feeding device 41 includes: a rotation-shaft portion 41a extending along CD direction; and a servomotor (not shown) serving as a drive source which drives and rotates the rotation-shaft portion 41a. The rotation-shaft portion 41a is inserted into a through hole disposed at the center of the material coil 4C. The rotation-shaft portion 41a is driven and rotate by the servomotor with supporting the material coil 4C, feeding the continuous sheet 4a of the back sheet from the material coil 4C.

The tension adjustment device 43 is for adjusting the tension value (N) of the continuous sheet 4a of the back sheet to the predetermined target value. In this example, a dancer-roll type one is employed. That is, the tension adjustment device 43 includes: a pair of stationary rolls 43RSu and 43RSd; a dancer roll 43RD; a measuring device (not shown); and a control section (not shown). The pair of stationary rolls 43RSu and 43RSd are arranged at respective fixed positions and are provided in a manner of being capable of rotating about the rotation shafts extending along CD direction. The dancer roll 43RD is guided between the pair of stationary rolls 43RSu and 43RSd in a manner of being capable of moving in a predetermined direction (the up-down direction in FIG. 5) and is provided in a manner of being capable of rotating about the rotation shaft extending along CD direction. The measuring device is a device (e.g., encoder) which detects the position of the dancer roll 43RD in the abovementioned predetermined direction and outputs positional information. The control section is a section to which the abovementioned positional information is inputted (e.g., a sequencer or a computer). To the dancer roll 43RD, an appropriate actuator (e.g., air cylinder; not shown) applies a load FD in a direction to increase the size of a loop (to be described later) in the abovementioned predetermined direction; the load FD corresponds to a target value of the tension value of the continuous sheet 4a. The continuous sheet 4a of the back sheet is wrapped around the pair of stationary rolls 43RSu and 43RSd and the dancer roll 43RD, forming a loop of the continuous sheet 4a. And, based on the abovementioned positional information that has been inputted, the abovementioned control section changes the feeding speed (mpm) of the rotation-shaft portion 41a so that the size of the loop is constant. For example, since the loop becomes smaller when the tension value is larger than the target value, in order to increase the size of the loop, correction is made in a direction to increase the instruction value of the feeding speed. In contrast, since the loop becomes larger when the tension value is smaller than the target value, in order to decrease the loop, correction is made in a direction to decrease the instruction value of the feeding speed. Such a correction is repeatedly made at a predetermined control cycle. Consequently, adjustment is made so that the tension value of the continuous sheet 4a at the position of the dancer roll 43RD is the abovementioned target value. It should be noted that, when the target value is need to change, the change can be achieved by changing the size of the abovementioned load FD by controlling the abovementioned actuator.

### (2) Back-Sheet Processing Line L4aK

In the back-sheet processing line L4aK, as shown in FIG. 5, as a processing for the continuous sheet 4a of the back sheet (corresponding to the processing or upstream processing), a plurality of single sheets of the leak-proof sheet 5 are bonded onto the continuous sheet 4a, at the product pitch P1 in the direction of transport. As another processing (corresponding to the downstream processing), a plurality of single pieces of the target tape are each bonded onto the continuous sheet 4a, at the product pitch P1 in the direction of transport. The bonding of the leak-proof sheet is performed by a leak-proof-sheet bonding device 45 arranged at a predetermined position P5 in the direction of transport in the back-sheet processing line L4aK. On the other hand, the bonding of the target tape 7 is performed by a target-tape bonding device 47 arranged at a position P7 downstream from the leak-proof-sheet bonding device 45.

The leak-proof-sheet bonding device 45 includes a rotating drum 45D (corresponding to the rotation body) and a servomotor (not shown). The rotating drum 45D is capable of rotating about a rotation shaft extending along CD direction while its outer circumferential surface 45Ds facing the transport path of the continuous sheet 4a of the back sheet. The servomotor serves as a drive source which drives and rotates the rotating drum 45D. On the outer circumferential surface 45Ds of the rotating drum 45D, suction force is produced due to suction, and therefore the plurality of the leak-proof sheets 5, 5,... are held on the outer circumferential surface 45Ds with arranged side-by-side at the product pitch P1y in the direction-of-rotation Dc45. Due to the rotation of the rotating drum 45D, each leak-proof sheet 5 is sent toward and passes through a facing position P4a1 (corresponding to the predetermined position) which faces the continuous sheet 4a, and the leak-proof sheet 5 is attached to the continuous sheet 4a with adhesive. Thus, the leak-proof sheet 5 is transferred from the outer circumferential surface 45Ds of the rotating drum 45D to the continuous sheet 4a.

The rotation operation of the rotating drum 45D is basically operated according to a synchronization signal. The synchronization signal is a signal composed of repeatedly outputted unit signals, and each unit signal is a rotational angle signal having a rotational angle value of 0° to 360°. In the processing systems L4a, L3a, L2 and L1a, each of the devices 36, 47, 112 and 116 (to be described later) including the bonding device 45 has its own systematic unit operation which the device should repeatedly perform for each to-be-diaper-1 unit part 1p of the continuous sheets 4a and 3a. Each unit operation is correlated to a unit signal on one-by-one basis under position control. For example, in the bonding device 45, its unit operation is a rotation operation of the product pitch P1, and this operation is correlated to a unit signal. That is, the synchronization signal is transmitted to a servomotor of the rotating drum 45D, and the rotating drum 45D is controlled its position so that, every time when each unit signal of synchronization signal is transmitted, the rotating drum 45D rotates by a rotational angle corresponding to the product pitch P1. More specifically, the servomotor of the rotating drum 45D is controlled its position so that a position where the leak-proof sheet 5 is sucked and held on the outer circumferential surface 45Ds is an instruction position in the direction-of-rotation Dc45 indicated by the synchronization signal. Basically, every time when each unit signal of the synchronization signal is outputted, the leak-proof sheets 5 which are placed at the product pitch P1 on the outer circumferential surface 45Ds are thus transported in the direction-of-rotation Dc45 by the product pitch P1.

The foregoing synchronization signal is generated by an appropriate control section (not shown) such as a sequencer or a computer. That is, the control section includes a processor and a memory, and the memory stores in advance a program for generating the synchronization signal. The processor reads and executes the abovementioned program in the memory, repeatedly generating a unit signal of the synchronization signal. However, the invention is not limited thereto. For example, the synchronization signal may be generated by an electric circuit. Or, the synchronization signal may be generated by detecting, using an appropriate detector, a unit operation to be conducted by other basic device. For example, the following configuration is also acceptable: an end-cutting device 116 which cuts the substrate 1a of the diaper and produces the diaper 1 is provided; on the cutter roll 116u of the end-cutting device 116 (see FIG. 4), a rotary encoder (not shown) is provided which detects the rotation operation of the cutter roll 116u; and the encoder outputs the foregoing unit signal in conjunction with the rotation operation of the cutter roll 116u every time when a single diaper 1 is cut out and produced from the substrate 1a.

The target-tape bonding device 47 also includes a rotating drum 47D and a servomotor (not shown). The rotating drum 47D is capable of rotating about a rotation shaft extending along CD direction while its outer circumferential surface 47Ds facing the transport path of the continuous sheet 4a of the back sheet. The servomotor serves as a drive source which drives and rotates the rotating drum 47D. On the outer circumferential surface 47Ds of the rotating drum 47D, suction force is produced due to suction, and therefore the plurality of target tapes 7, 7, ... are held on the outer circumferential surface 47Ds while arranged side-by-side at the product pitch P1 in the direction-of-rotation Dc47. Due to the rotation of the rotating drum 47D, each target tape 7 is sent toward and passes through a facing position P4a2 (corresponding to the predetermined position) which faces the continuous sheet 4a, and the target tape 7 is attached to the continuous sheet 4a with adhesive. Thus, the target tape 7 is transferred from the outer circumferential surface 47Ds of the rotating drum 47D to the continuous sheet 4a.

The rotation operation of the rotating drum 47D is basically operated according to a synchronization signal, in similar to the abovementioned leak-proof-sheet bonding device 45. That is, the servomotor of the rotating drum 47D is controlled its position so that a position where the target tape 7 is sucked and held on the outer circumferential surface 47Ds is an instruction position in the direction-of-rotation Dc47 indicated by the synchronization signal. Basically, every time when each unit signal of the synchronization signal is outputted, the target tapes 7 which are placed on the outer circumferential surface 47Ds at the product pitch P1 are transported in the direction-of-rotation Dc47 by the product pitch P1.

Meanwhile, the continuous sheet 4a of the back sheet is a sheet whose material is nonuniformity as mentioned in Technical Problem. Therefore, a problem described in Technical Problem can occur. That is, even if the back-sheet supply line L4aS transfers the continuous sheet 4a to the back-sheet processing line L4aK with a constant tension value (N), the amount of stretching of the continuous sheet 4a in the processing line L4aK can vary in a long cycle (see FIG. 1). This makes it difficult to perform the processing of the continuous sheet 4a in the processing line L4aK at a proper position in the continuous sheet 4a; that is, this makes it difficult to perform the abovementioned bonding of the leak-proof sheet 5 and the target tape 7 at a proper position in the continuous sheet 4a. Consequently, the bonded portion of the leak-proof sheet 5 and the bonded portion of the target tape 7 can be shifted in the direction of transport from their own proper positions in the continuous sheet 4a (hereinafter referred to as a target position).

In the present embodiment, the tension value at which the continuous sheet is transferred to the back-sheet processing line L4aK is adjusted as follow: detecting the bonded portion of the leak-proof sheet 5 (corresponding to the trace of the processing) in the continuous sheet 4a (corresponding to the detecting step); and based on that detection result, changing the target value of the abovementioned tension value (N) for the tension adjustment device 43 in the back-sheet supply line L4aS.

For example, in the case where the detection result indicates that the bonded portion of the leak-proof sheet 5 is located downstream in the direction of transport with respect to the target position in the continuous sheet 4a, the target value of the tension value is increased. This increases the tension value at the time of transferring the continuous sheet 4a of the back sheet to the back-sheet processing line L4aK. On the contrary, in the case where the detection result indicates that the bonded portion is located upstream in the direction of transport with respect to the target position, the target value of the tension value is decreased. This decreases the tension value at the time of transferring.

This makes it possible to suppress variation of the amount of the direction-of-transport stretching of the continuous sheet 4a which could cause in the back-sheet processing line L4aK. Consequently, it is possible to bond the leak-proof sheet 5 and the target tape 7 at their own target positions in the continuous sheet 4a.

Alignment of the position of bonded portion (hereinafter also referred to as the bonding position) with the target position is made by a detecting device 48 (FIG. 5) including an imaging device 48C (e.g., CCD camera) and a control section 48CN (e.g., a computer), and the detail is as follow.

First, as shown in FIG. 5, at a direction-of-transport position P48 (hereinafter also referred to as the imaging position P48) between the abovementioned merging position Pj and the position P7 where the target tape bonding device 47 is arranged, the imaging device 48C images the continuous sheet 4a and generates the image data of the continuous sheet 4a. Here, the imaging device 48C receives the abovementioned synchronization signal. Accordingly, an imaging operation is performed every time when the rotational angle value of the synchronization signal matches the specific rotational angle value which is prestored in a memory of the imaging device 48C. In the imaging operation, a captured image has a field of view (an angle of view) being capable of including at least one leak-proof sheet 5 in the direction of transport.

Then, the control section 48CN analyzes the image data transmitted from the imaging device 48C, obtaining information of the amount of displacement of the bonding position from the target position in the continuous sheet 4a of the back sheet. For example, the bonding position on the image data is obtained by means such as binarization process, and then calculated is the amount of the displacement the bonding position from the on-image-data target position which is prestored in a memory of the control section 48CN. And, the calculated becomes information of the amount of position displacement. The target position is a virtual position where, for example, the bonding position of the leak-proof sheet 5 should be located at the time of the abovementioned imaging, under the condition that the material of the continuous sheet 4a is uniform and that the sheet 4a is stretched uniformly throughout its total length. Such a target position can be known in advance by experiments with actual facilities.

Next, based on the information of the displacement amount of the position, the target value of the tension value for the tension adjustment device 43 is changed. For example, in the case where the abovementioned information of displacement amount indicates that "the bonding position is located downstream in the direction of transport with respect to the target position", the control section 48CN increases the target value of the tension value by an adjustment value (multiplied value of the displacement amount by a certain gain), making it larger than the current target value. On the other hand, in the case where the information indicates "the bonding position is located upstream in the direction of transport with respect to the target position", the control section 48CN decreases the target value of the tension value by an adjustment value (multiplied value of the displacement amount by a certain gain), making it smaller than the current target value.

It should be noted that, in this example, as shown in FIG. 5, the position P7 where the target-tape bonding device 47 performs bonding is located downstream in the direction of transport with respect to the position P5 where the leak-proof-sheet bonding device 45 bonds the leak-proof sheet 5, and the imaging position P48 is located further downstream. However, generally speaking, variation of the amount of stretching of the continuous sheet 4a of the back sheet accumulates at a position downstream in the direction of transport. Consequently, the displacement amount of the bonding position of the leak-proof sheet 5 from the target position in the continuous sheet 4a is conceivably tends to be more apparent at a position downstream in the direction of transport. Accordingly, in order to determine the displacement amount with high accuracy, as in the example of FIG. 5, the imaging position P48 is located downstream in the direction of transport with respect to the position P7 where the target-tape bonding device 47 bonds the target tape 7. It is more desirable that the imaging position P48 is located at a position immediately before the merging position Pj. Note that, the term "a position immediately before the merging position Pj" means any position within 2 m upstream from the merging position P; more desirably, any position within 1 m.

In some cases, based on the abovementioned information of the displacement amount of the position, rotation operation of the rotating drum 45D of the leak-proof-sheet bonding device 45 may be controlled. That is, concerning a timing at which the rotating drum 45D of the leak-proof-sheet bonding device 45 transfers the leak-proof sheet 5 to the facing position P4a1, the timing may be shifted from the timing indicated by the synchronization signal, based on the abovementioned information of the displacement amount of the position. More specifically, for example, in the case where the abovementioned displacement-amount information indicates "the bonding position of the leak-proof sheet 5 is located downstream in the direction of transport with respect to the target position", the control section 48CN shifts the instruction position toward upstream in the direction-of-rotation Dc45 by an adjustment value (multiplied value of the displacement amount by a certain gain) . On the other hand, in the case where the abovementioned displacement-amount information indicates "the bonding position of the leak-proof sheet 5 is located upstream in the direction of transport with respect to the target position", the control section 48CN shifts the instruction position toward downstream in the direction-of-rotation Dc45 by an adjustment value (multiplied value of the displacement amount by a certain gain).

It should be noted that, the abovementioned control for shifting the timing may be applied to the target-tape bonding device 47. That is, based on the abovementioned information of the displacement amount of the position, the timing at which the rotating drum 47D of the target-tape bonding device 47 transfers the target tape 7 to the facing position P4a2 may be shifted from the timing indicated by the synchronization signal.

In this example, in the back-sheet processing line L4aK, the continuous sheet 4a of the back sheet is transported in a predetermined transport path. However, as is apparent from FIGS. 4 and 5, the back-sheet processing line L4aK does not include a roll similar to the dancer roll 43RD which moves back and forth in a predetermined direction. Accordingly, the transport-path length of the processing line L4aK keeps constant. Concerning the bondings of the leak-proof sheet 5 and the target tape 7 which are performed based on the synchronization signal, this makes it possible to effectively prevent large shift of the bondings from the target position in the continuous sheet 4a.

Generally speaking, in the back-sheet processing line L4aK, the transport speed (mpm) of the continuous sheet 4a of the back sheet can be changed appropriately according to the production status of the manufacturing line LM. In this case, accompanying with change of the transport speed, the transport speed (mpm) of the transport speed in the back-sheet supply line L4aS can change. But, such a change of the transport speed has a risk of promoting variation of the amount of stretching of the abovementioned continuous sheet 4a. But, in this example, based on the abovementioned amount of displacement of position, the back-sheet supply line L4aS adjusts the tension value of the continuous sheet 4a at the time of transferring the sheet 4a to the back-sheet processing line L4aK, suppressing variation of the amount of stretching. Accordingly, even if the transport speed is changed as mentioned above, it is possible to bond the leak-proof sheet 5 and the target tape 7 at their own target positions in the continuous sheet 4a.

### <<< Top-Sheet Processing System L3a >>>

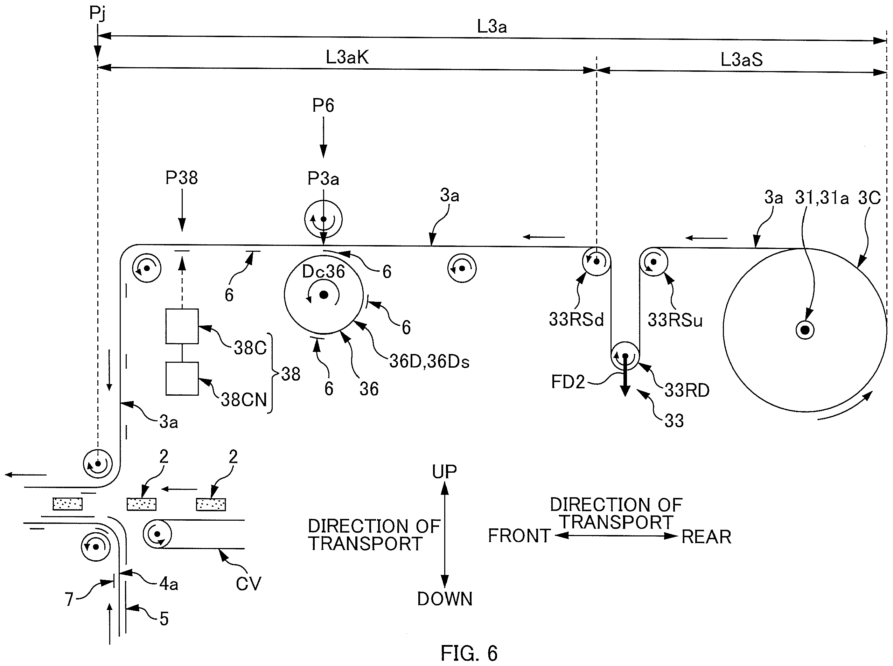

FIG. 6 is a schematic side view of the top-sheet processing system L3a.

The top-sheet processing system L3a includes: a top-sheet supply line L3aS; and a top-sheet processing line L3aK (corresponding to the second processing section) arranged downstream from the supply line L3aS in the direction of transport. In the top-sheet supply line L3aS, the continuous sheet 3a of the top sheet (corresponding to the second continuous sheet) is fed and supplied from the material coil 3C (corresponding to the second supplying step). In the top-sheet processing line L3aK, a certain process (corresponding to the second processing) is performed to the continuous sheet 3a of the top sheet which is supplied from the top-sheet supply line L3aS and transported in the direction of transport (corresponding to the second processing step).

### (1) Top-Sheet Supply Line L3aS

The top-sheet supply line L3aS includes: a feeding device 31 for feeding the continuous sheet 3a of the top sheet from the material coil 3C; and a tension adjustment device 33 which adjusts to a target value the direction-of-transport tension value (N) of the continuous sheet 3a which is fed by the device 31 and is being transported.

As shown in FIG. 6, the feeding device 31 includes: a rotation-shaft portion 31a extending along CD direction; and a servomotor (not shown) serving as a drive source which drives and rotates the rotation-shaft portion 31a. The rotation-shaft portion 31a is inserted into a through hole disposed at the center of the material coil 3C. The rotation-shaft portion 31a is driven and rotate by the servomotor with supporting the material coil 3C, feeding the continuous sheet 3a of the top sheet from the material coil 3C.

The tension adjustment device 33 is for adjusting the tension value (N) of the continuous sheet 3a of the top sheet to the predetermined target value. In this example, a dancer-roll type one is employed similarly to the abovementioned back-sheet supply line L4aS. That is, the tension adjustment device 31 includes: a pair of stationary rolls 33RSu and 33RSd; a dancer roll 33RD; an appropriate actuator (not shown); a measuring device (not shown); and a control section (not shown). The pair of stationary rolls 33RSu and 33RSd are arranged at respective fixed positions and are provided in a manner of being capable of rotating about the rotation shafts extending along CD direction. The dancer roll 33RD is guided between the pair of stationary rolls 33RSu and 33RSd in a manner of being capable of moving in a predetermined direction (the up-down direction in FIG. 6) and is provided in a manner of being capable of rotating about the rotation shaft extending along CD direction. The actuator (e.g., air cylinder) applies, to the dancer roll 33RD, a load FD2 in a direction to increase the size of a loop (to be described later) in the abovementioned predetermined direction; the load FD2 corresponds to the target value of the tension value of the continuous sheet 3a. The measuring device is a device (e.g., encoder) which detects the position of the dancer roll 33RD in the abovementioned predetermined direction and outputs positional information. The control section is a section to which the abovementioned positional information is inputted (e.g., a sequencer or a computer). The continuous sheet 3a of the top sheet is wrapped around the pair of stationary rolls 33RSu and 33RSd and the dancer roll 33RD, forming a loop of the continuous sheet 3a. Accordingly, in similar to the abovementioned back-sheet supply line L4aS, based on the abovementioned positional information that has been inputted, the abovementioned control section changes the feeding speed (mpm) of the rotation-shaft portion 31a so that the size of the loop is constant. Consequently, adjustment is made so that the tension value of the continuous sheet 3a at the position of the dancer roll 33RD is the abovementioned target value. Note that, when the target value is needed to change, the change can be achieved by changing the size of the abovementioned load FD2 by controlling the abovementioned actuator.

### (2) Top-Sheet Processing Line L3aK

In the top-sheet processing line L3aK, as shown in FIG. 6, as a processing for the continuous sheet 3a of the top sheet (corresponding to the second processing), a pair of fastening-tape members 6 and 6 located side-by-side in CD direction is bonded onto the continuous sheet 3a, at the product pitch P1 in the direction of transport. The bonding is performed by a fastening-tape-member bonding device 36 arranged at a predetermined position P6 in the direction of transport in the top-sheet processing line L3aK.

The fastening-tape-member bonding device 36 includes a rotating drum 36D and a servomotor (not shown). The rotating drum 36D is capable of rotating about a rotation shaft extending along CD direction while its outer circumferential surface 36Ds facing the transport path of the continuous sheet 3a of the top sheet. The servomotor serves as a drive source which drives and rotates the rotating drum 36D. On the outer circumferential surface 36Ds of the rotating drum 36D, suction force is produced due to suction, and therefore a pair of fastening-tape members 6 and 6 located side-by-side in CD direction are held on the outer circumferential surface 36Ds while arranged side-by-side at the product pitch P1 in the direction-of-rotation Dc36. Due to the rotation of the rotating drum 36D, each pair of fastening-tape members 6 and 6 is sent toward and passes through a facing position P3a which faces the continuous sheet 3a, and the pair of fastening-tape members 6 and 6 is attached to the continuous sheet 3a with adhesive. Thus, the pair of fastening-tape members 6 and 6 is transferred from the outer circumferential surface 36Ds of the rotating drum 36D to the continuous sheet 3a.

The rotation operation of the rotating drum 36D is basically operated according to a synchronization signal, in similar to the abovementioned leak-proof-sheet bonding device 45. That is, the servomotor of the rotating drum 36D is controlled its position so that a position where a pair of fastening-tape members 6 and 6 are sucked and held on the outer circumferential surface 36Ds is an instruction position in the direction-of-rotation Dc 36 indicated by synchronization signal. Basically, every time when each unit signal of the synchronization signal is outputted, pairs of fastening-tape members 6 and 6 which are placed on the outer circumferential surface 36Ds at the product pitch P1 are transported in the direction-of-rotation Dc 36 by the product pitch P1.

Meanwhile, the continuous sheet 3a of the top sheet is a sheet whose material is nonuniformity as mentioned in Technical Problem. Therefore, a problem described in Technical Problem can occur. That is, even if the top-sheet supply line L3aS transfers the continuous sheet 3a to the top-sheet processing line L3aK at a constant tension value (N), the amount of stretching of the continuous sheet 3a in the processing line L3aK can vary in a long cycle (see FIG. 1). This makes it difficult to perform the processing of the continuous sheet 3a in the processing line L3aK at a proper position in the continuous sheet 3a; that is, this makes it difficult to perform the abovementioned bonding of the pair of fastening-tape members 6 and 6 at a proper position in the continuous sheet 3a. Consequently, the bonding position of the pair of fastening-tape members 6 and 6 can be shifted in the direction of transport from its proper position in the continuous sheet 3a (hereinafter referred to as a target position).

At this stage, the tension value at which the continuous sheet 3a of the top sheet is transferred to the top-sheet processing line L3aK is adjusted as follow: detecting bonded portions of the pair of fastening-tape members 6 and 6 (corresponding to the trace of the second processing) in the continuous sheet 3a (corresponding to the second detecting step); and based on that detection result (corresponding to the second detection result), changing the target value of the abovementioned tension value (N) for the tension adjustment device 33 in the top-sheet supply line L3aK.

For example, in the case where the detection result indicates that the bonded portions of the pair of fastening-tape members 6 and 6 are located downstream in the direction of transport with respect to the target positions in the continuous sheet 3a, the target value of the tension value is increased. This increases the tension value at the time of transferring the continuous sheet 3a of the top sheet to the top-sheet processing line L3aK. On the contrary, in the case where the detection result indicates that the bonded portions are located upstream in the direction of transport with respect to the target position, the target value of the tension value is decreased. This decreases the tension value at the time of transferring.

This makes it possible to suppress variation of the amount of the direction-of-transport stretching of the continuous sheet 3a which could cause in the top-sheet processing line L3aK. Consequently, it is possible to bond the pair of fastening-tape members 6 and 6 at their target positions in the continuous sheet 3a.

In this case, at the abovementioned merging position Pj, the continuous sheet 3a of the top sheet and the continuous sheet 4a of the back sheet are bonded while suppressing variation of the amount of stretching of both sheets. This makes it possible to prevent a shape-related or size-related trouble such as warping of diaper 1 or size irregularity, which could be caused in the case where these continuous sheets 3a and 4a are bonded under the condition that the variation is not suppressed.

Alignment of the position of the bonded portion (hereinafter also referred to as the bonding position) with the target position is made by a detecting device 38 (FIG. 6) including an imaging device 38C (e.g., CCD camera) and a control section 38CN (e.g., a computer). The concrete method is the same as that in the case of the abovementioned back-sheet supply line L3aS. Accordingly, the description thereof is omitted.

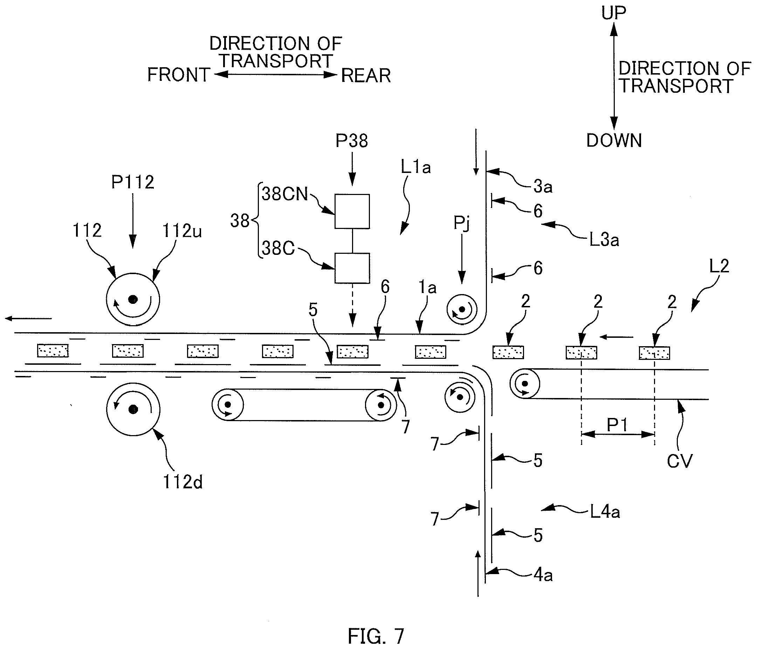

In some cases, as shown in FIG. 7, the imaging position P38 of the imaging device 38C may be located downstream in the direction of transport with respect to the abovementioned merging position Pj. That is, the imaging position P38 may be located in the substrate processing system L1a. In this case, the imaging device 38C can generate not only the image data of the continuous sheet 3a of the top sheet, but also the image data of the abovementioned the continuous sheet 4a of the back sheet. Accordingly, it is possible to omit the detecting device 48 (FIG. 6) arranged in the abovementioned the back-sheet processing line L4aK. In the case where the substrate 1a is imaged using transmitted light which is transmitted the substrate 1a of the diaper in the thickness direction, one image data generated in a single imaging operation can contain the following information: the information of the bonding position of the leak-proof sheet 5 in the continuous sheet 4a of the back sheet; and the information of bonding positions of the pair of fastening-tape members 6 and 6 in the continuous sheet 3a of the top sheet. This makes it possible to reduce the number of imaging operations, making it possible to reduce load of the imaging device 38C.

### <<< Absorbent-Body Producing System L2>>>

As shown in FIG. 4, the absorbent-body producing system L2 includes a transport device CV such as a belt conveyer. The transport device CV works in conjunction with the abovementioned synchronization signal, transporting the absorbent bodies 2, 2,... to the merging position Pj at the product pitch P1 in the direction of transport.

### <<< Substrate Processing System L1a >>>

With reference to FIG. 4, the substrate processing system L1a is located downstream with respect to the merging position Pj as mentioned above. In the substrate processing system L1a, a leg-opening cutting device 112 and an end-cutting device 116 are arranged in this order from upstream to downstream in the direction of transport. First, the cutting device 112 cuts out both CD-direction side portions of the substrate 1a of the diaper which is transported from the merging position Pj, forming the pair of leg openings 1LH and 1LH in the substrate 1a, which are lined side-by-side located in CD direction. Next, the cutting device 116 cuts the substrate 1a at a position Plac which is between the absorbent bodies 2 and 2 adjacent in the direction of transport, and a downstream end portion is cut and separated from the substrate 1a. Thus, a diaper 1 shown in FIG. 2A is produced.

The leg-opening cutting device 112 includes: a pair of upper and lower rolls 112u and 112d provided in a manner of being capable of rotating about the rotation shaft extending along CD direction; and servomotors (not shown) that drives and rotates the pair of rolls 112u and 112d as drive sources. The upper roll 112u is a cutter roll. That is, on the outer circumferential surface of the upper roll 112u, cutter blades (not shown) are provided at every predetermined angle in the direction-of-rotation Dc112 and the cutter blades each have a curved shape corresponding to the leg opening 1LH of the diaper 1. On the other hand, lower roll 112d is an anvil roll whose outer circumferential surface receives the cutter blade. The rotation operations of these rolls 112u and 112d are basically operated according to the abovementioned synchronization signal. That is, basically, a servomotor is controlled its position so that a position of the cutter blade is an instruction position in the direction-of-rotation Dc112 indicated by the synchronization signal. Accordingly, every time when each unit signal of the synchronization signal is outputted, the cutter blade rotates so as to face a pair of leg-opening-lLH-to-be-formed portions of the substrate 1a of the diaper, that is, so as to face portions of the substrate 1a which located outside the absorbent body 2 in CD direction. Consequently, leg openings 1LH and 1LH are formed in the substrate 1a.

The end-cutting device 116 includes: a pair of upper and lower rolls 116u and 116d provided in a manner of being capable of rotating about the rotation shaft extending along CD direction; and servomotors (not shown) that drives and rotates the pair of rolls 116u and 116d as drive sources. The upper roll 116u is a cutter roll. That is, on the outer circumferential surface of the upper roll 116u, cutter blades (not shown) are provided at every predetermined angle in the direction-of-rotation Dc116, and the cutter blades each have straight shape extending along CD direction. On the other hand, the lower roll 116d is an anvil roll whose outer circumferential surface receives the cutter blade. The rotation operations of these rolls 116u and 116d are basically operated according to the abovementioned synchronization signal. That is, basically, a servomotor is controlled its position so that a position of the cutter blade is an instruction position in the direction-of-rotation Dc116 indicated by the synchronization signal. Accordingly, every time when each unit signal of the synchronization signal is outputted, the cutter blade rotates so as to face a position P1ac where to be cut the substrate 1a of the diaper, that is, so as to face the position P1ac in the substrate 1a between the absorbent bodies 2 and 2 adjacent in the direction of transport. Consequently, a downstream end portion is cut and separated from the substrate 1a, producing a diaper 1.

### === Modified Example ===

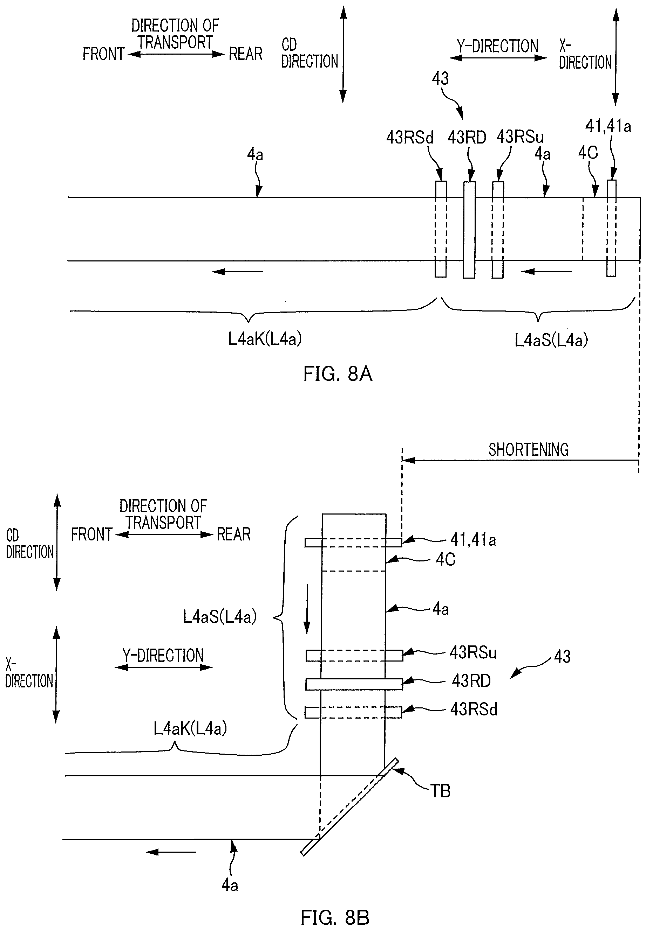

As shown in the schematic plan view of FIG. 8A, a direction along CD direction when the manufacturing line LM is viewed from above is defined as "X-direction", and a direction perpendicular to CD direction is defined as "Y-direction". In the foregoing embodiments, as shown in FIG. 8A, a direction of feeding of the continuous sheet 4a by the feeding device 41 in the back-sheet supply line L4aS is Y-direction, and also the direction of transport of the continuous sheet 4a in the back-sheet processing line L4aK is Y-direction in the same direction as the foregoing direction of feeding the continuous sheet 4a. However, the invention is not limited thereto. That is, the configuration shown in the modified example of FIG. 8B is also acceptable; that is, the direction of feeding in the back-sheet supply line L4aS may extend mainly along X-direction, and the direction of transport in the back-sheet processing line L4aK may extend mainly along Y-direction.

With such a configuration, as shown in FIG. 8B, the position where the rotation-shaft portion 41a of the feeding device 41 is arranged can be located at a position different from the back-sheet processing line L4aK in X-direction (CD direction). This can reduce the total length of the manufacturing line LM in Y-direction, making it possible to downsize the manufacturing line LM.

As shown in FIG. 8B, arranging the rotation-shaft portion 41a of the feeding device 41 along Y-direction realizes that the direction of feeding of the continuous sheet 4a is X-direction (CD direction) . A turn bar TB (corresponding to the direction-of-transport changing member) enables that the direction of transport of the continuous sheet 4a changes from X-direction to Y-direction. The turn bar TB is, for example, a round bar having a diameter of 25.4 mm. And, the turn bar TB is arranged in immovable and unrotatable manner with the longitudinal direction thereof being inclined by 45° with respect to both of X-direction and Y-direction. Accordingly, the continuous sheet 4a of the back sheet is wrapped around the turn bar TB, and as a result the direction of transport of the continuous sheet 4a changes from X-direction to Y-direction.

However, providing the turn bar TB makes it easier to cause such a trouble that, when tension variation of the continuous sheet 4a of the back sheet occurs at the position of the turn bar TB, the continuous sheet 4a is hooked to the unrotating, turn bar TB or sliding resistance between the continuous sheet 4a and the turn bar TB becomes excessively large. Accordingly, it is necessary to set the tension value to the optimal one for making the turn bar TB pass. In some cases, the foregoing tension value (N) is different from an optimal tension value (N) at the time of transferring the continuous sheet 4a to the back-sheet processing line L4aK. For example, there is a case where the former optimal tension value is smaller than the latter optimal tension value, or vice versa.

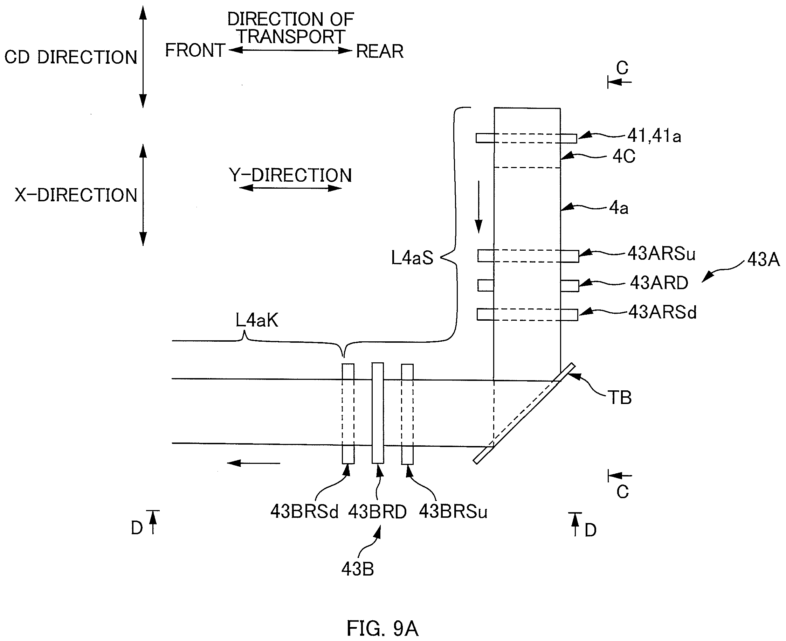

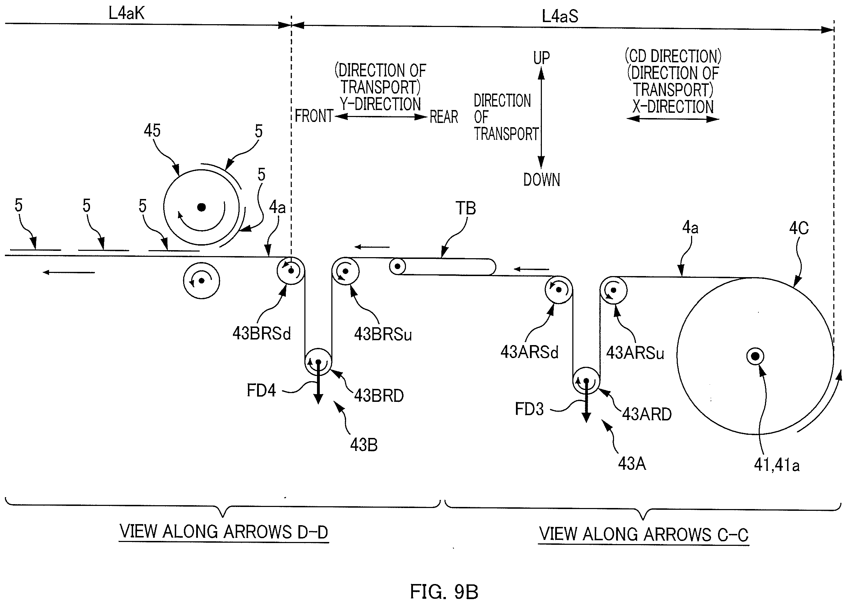

In such a case, it is preferable that two tension adjustment devices 43A and 43B are provided. FIGS. 9A and 9B are diagram showing examples thereof. FIG. 9A is a schematic plan view. FIG. 9B is a schematic side view in which a view along arrows C-C in FIG. 9A and a view along arrows D-D in FIG. 9A are connected and illustrated in a plane.

As shown in FIGS. 9A and 9B, in this example, one tension adjustment device 43A (corresponding to the second tension adjustment device) is provided between the feeding device 31 and the turn bar TB, and also another tension adjustment device 43B (corresponding to the first tension adjustment device) is provided between the turn bar TB and the back-sheet processing line L4aK. This makes it possible to adjust each tension value individually.

As shown in FIG. 9B, the tension adjustment device 43A adjusts a tension value at the position of the turn bar TB. The configuration of the tension adjustment device 43A is substantially the same as that of the abovementioned tension adjustment device 43 which is a dancer-roll type. That is, the tension adjustment device 43A includes: a pair of stationary rolls 43ARSu and 43ARSd; a dancer roll 43ARD; an appropriate actuator (not shown); a measuring device (not shown); and a control section (not shown). The pair of stationary rolls 43ARSu and 43ARSd are arranged at respective fixed positions and provided in a manner of being capable of rotating about the rotation shaft extending along Y-direction. The dancer roll 43ARD is guided between the pair of stationary rolls 43ARSu and 43ARSd in a manner of being capable of moving in the predetermined direction (up-down direction in FIG. 9B) and provided in a manner of being capable of rotating about the rotation shaft extending along Y-direction. The actuator is air cylinder or the like, and applies load FD3 to the dancer roll 43ARD in a direction to increase the size of a loop (to be described later) in the abovementioned predetermined direction; the load FD3 corresponds to the target value (corresponding to a predetermined value) of the tension value of the continuous sheet 4a. The measuring device (e.g., an encoder) detects a position of the dancer roll 43ARD in the predetermined direction and outputs positional information. The control section is a section to which the abovementioned positional information is inputted (e.g., a sequencer or a computer) . The continuous sheet 4a of the back sheet is wrapped around the pair of stationary rolls 43ARSu and 43ARSd and the dancer roll 43ARD, forming a loop of the continuous sheet 4a. Accordingly, based on the abovementioned positional information that has been inputted, the abovementioned control section changes the feeding speed (mpm) of the rotation-shaft portion 41a so that the size of the loop is constant. Consequently, adjustment is made so that the tension value (N) of the continuous sheet 4a at the position of the dancer roll 43ARD is the predetermined target value, and also so that the tension value (N) at the position of the turn bar TB is the predetermined target value.

On the other hand, as shown in FIG. 9B, the tension adjustment device 43B adjusts a tension value at the time of transferring the continuous sheet 4a to the back-sheet processing line L4aK. The device 43B is also a dancer-roll type. That is, the tension adjustment device 43B includes: a pair of stationary rolls 43BRSu and 43BRSd; a dancer roll 43BRD; an appropriate actuator (not shown); a measuring device (not shown); and a control section (not shown). The pair of stationary rolls 43BRSu and 43BRSd are arranged at respective fixed positions and provided in a manner of being capable of rotating about the rotation shaft extending along X-direction. The dancer roll 43BRD is guided between the pair of stationary rolls 43BRSu and 43BRSd in a manner of being capable of moving in the predetermined direction (up-down direction in FIG. 9B) and provided in a manner of being capable of rotating about the rotation shaft extending along X-direction. The actuator is air cylinder or the like, and applies load FD4 to the dancer roll 43BRD in a direction to increase the size of a loop (to be described later) in the abovementioned predetermined direction; the load FD4 corresponds to the target value of the tension value of the continuous sheet 4a. The measuring device (e.g., an encoder) detects a position of the dancer roll 43BRD in the predetermined direction and outputs positional information. The control section is a section to which the abovementioned positional information is inputted (e.g., a sequencer or a computer). The continuous sheet 4a of the back sheet is wrapped around the pair of stationary rolls 43BRSu and 43BRSd and the dancer roll 43BRD, forming a loop of the continuous sheet 4a. Of the pair of stationary rolls 43BRSu and 43BRSd, the stationary roll 43BRSu is located upstream in the direction of transport; in other words, the stationary roll 43BRSu is located upstream in the direction of transport with respect to the dancer roll 43BRD. The stationary roll 43BRSu serves as a driving roll (hereinafter also referred to as a stationary driving roll 43BRSu) which is rotated by a servomotor as a drive source. Accordingly, based on the abovementioned positional information that has been inputted, the abovementioned control section changes the rotation speed (mpm) of the abovementioned stationary driving roll 43BRSu so that the size of the loop is constant. Consequently, adjustment is made so that the tension value of the continuous sheet 4a at the position of the dancer roll 43BRD is the predetermined target value.

Further, similarly to the abovementioned embodiment, the target value of the tension value (N) for the tension adjustment device 43B is changed based on the detection result of the bonded portion of the leak-proof sheet 5 in the continuous sheet 4a of the back sheet. For example, in the case where the detection result of the detecting device 48 indicates that the bonded portion of the leak-proof sheet 5 is located downstream in the direction of transport with respect to the target position in the continuous sheet 4a, the control section 48CN of the detecting device 48 increases the target value of the tension value. This increases the tension value at the time of transferring the continuous sheet 4a of the back sheet to the back-sheet processing line L4aK. On the contrary, in the case where the detection result indicates that the bonded portion is located upstream in the direction of transport with respect to the target position, the control section 48CN decreases the target value of the tension value. This decreases the tension value at the time of transferring. This makes it possible to suppress variation of the amount of the direction-of-transport stretching of the continuous sheet 4a which could cause in the back-sheet processing line L4aK. Consequently, it is possible to bond the leak-proof sheet 5 and the target tape 7 at their own target positions in the continuous sheet 4a. It should be noted that, the target value can be changed by changing the magnitude of the load FD4 by controlling the actuator.

As is apparent from FIG. 9B, in this example, the tension adjustment device 43B is arranged close to the back-sheet processing line L4aK. This makes it possible to transfer to the processing line L4aK the continuous sheet 4a whose tension value is adjusted.

It goes without saying that such a configuration of the modified example, that is, a configuration including the turn bar TB and two tension adjustment devices 43A and 43B may be applied to the top-sheet supply line L3aS of FIG. 6. Conceivably, it is sufficiently possible to reach the configuration of such a case based on the foregoing description. Therefore, description thereof is omitted.

### === Other Embodiments ===

While the embodiment according to the invention are described, the above embodiment of the invention are for facilitating understanding of the invention, and are not limiting of the invention. The invention can of course be altered and improved without departing from the gist thereof, and equivalents are intended to be embraced therein. For example, the invention can be altered as described below.

In the foregoing embodiments, an absorbent article is exemplified by a so-called tape-type disposable diaper 1. However, the invention is not limited thereto. For example, a pull-on disposable diaper may be employed. As a pull-on disposable diaper, a so-called 3-piece diaper may be employed in which a liquid-permeable absorbent main body (a sheet-like member having the absorbent body 2 therein) bridges between a front band member and a back band member when the diaper is open, or a so-called 2-piece diaper may be employed in which an absorbent main body is placed on the skin-side surface of an exterior sheet having a substantially hour-glass shape when the diaper is open. In addition, the absorbent article is not limited to the disposable diaper 1. That is, it may be any article that absorbs excreted fluid from the wearer. For example, the absorbent article may be a sanitary napkin, a urine absorbing pad, or the like.

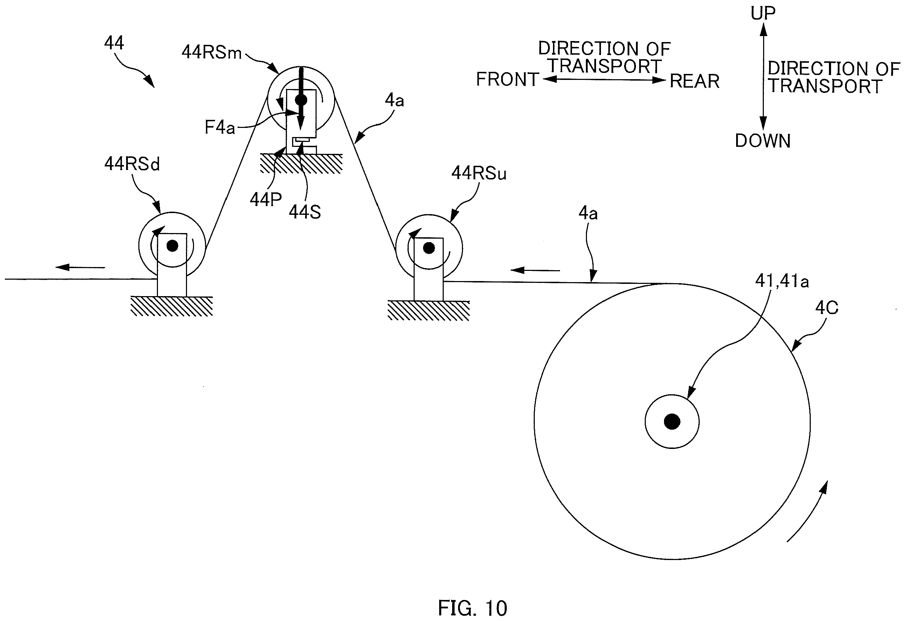

In the foregoing embodiments, as an example of the tension adjustment device 43, a dancer-roll type one is provided as shown in FIG. 5. However, the invention is not limited thereto. That is, a device may be employed which measures the tension value of the continuous sheet 4a and which can change the feeding speed (mpm) of the feeding device 41 so that the tension value becomes the target value. FIG. 10 is a diagram illustrating a tension adjustment device 44, which is an example thereof. First, instead of the dancer roll 43RD and the pair of stationary rolls 43RSu and 43RSd located upstream and downstream therefrom shown in FIG. 5, the tension adjustment device 44 of FIG. 10 includes three rolls 44RSu, 44RSm and 44RSd at fixed positions in the direction of transport. The three rolls 44RSu, 44RSm and 44RSd are supported in a manner of being capable of rotating about the rotation shaft extending along CD direction. Of the three rolls 44RSu, 44RSm and 44RSd, the center roll 44RSm is supported by a portion 44P, in which a sensor 44S such as a strain gauge is provided. The abovementioned sensor 44S outputs in real time a value depending on force F4a which is applied by the continuous sheet 4a to the center roll 44RSm while the continuous sheet 4a being wrapped around the three roll 44RSu, 44RSm and 44RSd in a form of mountain. In addition, the value outputted by the sensor 44S is converted to a tension value in real time by an appropriate converter (not shown). The feeding speed (mpm) of the feeding device 41 may be changed so that the tension value becomes the abovementioned target value.

It should be noted that, the foregoing tension adjustment device 44 may be used instead of the tension adjustment device 33 of the top-sheet supply line L3aS in FIG. 6, or may be used instead of the tension adjustment devices 43A and 43B of the modified example shown in FIG. 9B. However, in the case where it is used instead of the tension adjustment device 43B of FIG. 9B, the rotation speed (mpm) of the stationary driving roll 43BRSu is changed so that the tension value measured by the sensor 44S becomes the abovementioned target value.

In the foregoing embodiments, the processing which is performed to a to-be-absorbent article portion of the continuous sheet at an interval in the direction of transport is exemplified by the following processes: bonding of the leak-proof sheet 5 to the continuous sheet 4a of the back sheet; bonding of the target tape 7 to the continuous sheet 4a; and bonding of the pair of fastening-tape members 6 and 6 to the continuous sheet 3a of the top sheet. However, the processing is not limited thereto. For example, the processing may be applying adhesive onto the continuous sheets 4a and 3a at an interval in the direction of transport, or may be intermittently-pressing, welding, cutting, or the like.

In the foregoing embodiments, as shown in FIG. 5, the tension adjustment device 43 is exemplified by a device including: the pair of stationary rolls 43RSu and 43RSd arranged at respective fixed positions and provided in a manner of being capable of rotating about the rotation shaft extending along CD direction; the dancer roll 43RD guided between the pair of stationary rolls 43RSu and 43RSd in a manner of being capable of moving in the predetermined direction(up-down direction in FIG. 4) and provided in a manner of being capable of rotating about the rotation shaft extending along CD direction; the actuator applies load FD to the dancer roll 43RD in a direction to increase the size of a loop in the predetermined direction, the load FD corresponding to the target value of the tension value of the continuous sheet 4a; the measuring device that detects the position of the dancer roll 43RD in the predetermined direction and outputs positional information; and the control section to which the positional information is inputted. The tension adjustment device 43 changes the foregoing target value of the tension value based on the detection result of the bonded portion of the leak-proof sheet 5 in the continuous sheet 4a of the back sheet, adjusting the tension at the time of transferring the continuous sheet 4a to the back-sheet processing line L4aK. However, the invention is not limited thereto.

That is, in the case where, of the pair of stationary rolls 43RSu and 43RSd shown in FIG. 5, the stationary roll 43RSd located downstream in the direction of transport is a driving roll which is driven and rotated by a servomotor as a drive source, the rotation speed (mpm) of the driving roll 43RSd may be changed based on the abovementioned detection result instead of changing the target value of the tension value.

For example, in the case where the detection result indicates that the bonded portion of the leak-proof sheet 5 is located downstream in the direction of transport with respect to the target position in the continuous sheet 4a, the rotation speed (mpm) of the driving roll 43RSd is decreased smaller than the current value. This increases the tension value at the time of transferring the continuous sheet 4a to the back-sheet processing line L4aK. On the contrary, in the case where the detection result indicates that the bonded portion is located upstream in the direction of transport with respect to the target position, the rotation speed (mpm) is increased larger than the current value. This decreases the tension value at the time of transferring.

This makes it possible to suppress variation of the amount of the direction-of-transport stretching of the continuous sheet 4a which could cause in the back-sheet processing line L4aK. Consequently, it is possible to bond the leak-proof sheet 5 and the target tape 7 at their own target positions in the continuous sheet 4a.

It should be noted that, the same is applied to the tension adjustment device 33 in the top-sheet supply line L3aS shown in FIG. 6.

### [Reference Signs List]

1 disposable diaper (absorbent article), 1LH leg opening, 1a substrate of diaper,
   1f front part, 1b back part, 1p portion which is to be a diaper (portion which is to be an absorbent article),
2 absorbent body,
3 top sheet, 3a continuous sheet of top sheet (second continuous sheet),
3C material coil,
4 back sheet, 4a continuous sheet of back sheet (continuous sheet),
4C material coil,
5 leak-proof sheet,
6 fastening-tape member, 6m male member, 7 target tape,
31 feeding device, 31a rotation-shaft portion,
33 tension adjustment device, 33RD dancer roll,
33RSu stationary roll, 33RSd stationary roll,
36 fastening-tape-member bonding device, 36D rotating drum, 36Ds outer circumferential surface,
38 detecting device, 38C imaging device, 38CN control section,
41 feeding device, 41a rotation-shaft portion,
43 tension adjustment device, 43RD dancer roll,
43RSu stationary roll, 43RSd stationary roll,
43A tension adjustment device(second tension adjustment device), 43ARD dancer roll,
43ARSu stationary roll, 43ARSd stationary roll,
43B tension adjustment device(first tension adjustment device), 43BRD dancer roll,
43BRSu stationary roll, 43BRSd stationary roll,
44 tension adjustment device, 44RSu roll, 44RSm roll, 44RSd roll,
44P portion, 44S sensor,
45 leak-proof-sheet bonding device, 45D rotating drum(rotation body), 45Ds outer circumferential surface,
47 target-tape bonding device, 47D rotating drum, 47Ds outer circumferential surface,
48 detecting device, 48C imaging device, 48CN control section,
112 leg-opening cutting device, 112u upper roll, 112d lower roll,
116 end-cutting device, 116u upper roll, 116d lower roll,
TB turn bar (direction-of-transport changing member),
CV transport device,
L1a substrate processing system,
L2 absorbent-body producing system,
L3a top-sheet processing system, L3aK top-sheet processing line(second processing section),
L3aS top-sheet supply line,
L4a back-sheet processing system, L4aK back-sheet processing line(processing section),
L4aS back-sheet supply line(supplying section),
LM manufacturing line,
P1ac position,
P3a facing position,
P4a1 facing position (predetermined position), P4a2 facing position (predetermined position),
P5 predetermined position, P6 predetermined position, P7 predetermined position,
Pj merging position, P38 imaging position, P48 imaging position,
P112 predetermined position, P116 position,

## Claims

1. A method for manufacturing a sheet-like member using a continuous sheet (4a), the sheet-like member associated with an absorbent article (1),
the method comprising:
a supplying step (L4aS) of supplying the continuous sheet,
the supplying step being performed by transferring the continuous sheet to a processing section by transporting the continuous sheet in a direction of transport,
the direction of transport being a direction in which the continuous sheet continues;
a processing step (L4aK) of processing a portion of the continuous sheet by the processing section at an interval in the direction of transport,
the portion being to be the absorbent article,
the processing step being performed at a predetermined position in the direction of transport;
a detecting step (48) of detecting a trace of the processing left on the continuous sheet and outputting a detection result,
the supplying step including adjusting a direction-of-transport tension value (43, 43A, 43B, 44) of the continuous sheet based on the detection result,
the direction-of-transport tension value of the continuous sheet being a tension value at a time of transferring the continuous sheet to the processing section, wherein
if the detection result indicates that the trace of the processing is located downstream in the direction of transport with respect to a target position in the continuous sheet,
the tension value is increased, and
if the detection result indicates that the trace of the processing is located upstream in the direction of transport with respect to the target position,
the tension value is decreased, wherein
the processing is defined as an upstream processing (45),
another processing is defined as a downstream processing (47),
the downstream processing is performed, at an interval in the direction of transport, at a position (P7) downstream in the direction of transport with respect to a position (P5) where the upstream processing is performed,
a portion of the continuous sheet that is to be the absorbent article is subject to the downstream processing, and
the trace of the processing is detected at a position (P48) downstream with respect to the position where the downstream processing is performed.

2. A method for manufacturing a sheet-like member associated with an absorbent article according to claim 1, wherein
in the processing section, the continuous sheet is transported in a predetermined transport path, and
a direction-of-transport length of the transport path keeps constant.

3. A method for manufacturing a sheet-like member associated with an absorbent article according to any one of claims 1 to 2, wherein
in the supplying step, a feeding device that feeds the continuous sheet from a material coil is used,
in the feeding device, the continuous sheet is fed and transported while a direction of feeding of the continuous sheet being X-direction as viewed from above,
in the processing section, the continuous sheet is transported while the direction of transport of the continuous sheet being Y-direction as viewed from above, the Y-direction intersecting the X-direction,
a bar-shaped direction-of-transport changing member is arranged at a position in the direction of transport between the feeding device and the processing section,
the direction of transport of the continuous sheet changes from the X-direction to the Y-direction by wrapping the continuous sheet around the direction-of-transport changing member, and
the tension adjustment device is arranged at a position in the direction of transport between the direction-of-transport changing member and the processing section.

4. A method for manufacturing a sheet-like member associated with an absorbent article according to claim 3, wherein
the tension adjustment device is defined as a first tension adjustment device,
a second tension adjustment device is arranged between the feeding device and the direction-of-transport changing member in the direction of transport, and
the second tension adjustment device is for controlling a feeding operation of the feeding device so that a tension value of the continuous sheet that has been fed from the feeding device becomes a predetermined value.

5. A method for manufacturing a sheet-like member associated with an absorbent article according to any one of claims 1 to 4, wherein in the processing section, a transport speed at which the continuous sheet is transported in the direction of transport is changed.

6. A method for manufacturing a sheet-like member associated with an absorbent article according to any one of claims 1 to 5, wherein a timing at which the processing is performed in the processing step is shifted based on the detection result.

7. A method for manufacturing a sheet-like member associated with an absorbent article according to any one of claims 1 to 6, wherein
a first continuous sheet is the continuous sheet,
a second continuous sheet is a continuous sheet different from the first continuous sheet, and
at a merging position downstream in the direction of transport with respect to a position where the processing is performed,
the second continuous sheet is transferred and bonded with the first continuous sheet.

8. A method for manufacturing a sheet-like member associated with an absorbent article according to claim 7, wherein
the method further comprises:
a second supplying step of supplying the second continuous sheet,
the second supplying step being performed by transferring the second continuous sheet to a second processing section by transporting the second continuous sheet in a direction of transport,
the direction of transport being a direction in which the second continuous sheet continues;
a second processing step of performing a second processing to a portion of the second continuous sheet by the second processing section at an interval in the direction of transport,
the portion being to be the absorbent article,
the second processing step being performed at a predetermined position in the direction of transport; and
a second detecting step of detecting a trace of the second processing left on the second continuous sheet and outputting a second detection result, the second supplying step includes adjusting a direction-of-transport tension value of the continuous sheet based on the second detection result,
the direction-of-transport tension value of the continuous sheet being a tension value at the time of transferring the continuous sheet to the second processing section, and
the merging position is located downstream in the direction of transport with respect to a position where the second processing is performed.

9. A method for manufacturing a sheet-like member associated with an absorbent article according to claim 8, wherein
a trace of the processing left on the first continuous sheet is detected at a position downstream in the direction of transport with respect to the merging position.

10. An apparatus for manufacturing a sheet-like member using a continuous sheet (4a), the sheet-like member associated with an absorbent article (1),
the apparatus comprising:
a supplying section (L4aS) that supplies the continuous sheet by transferring the continuous sheet to a processing section (L4aK) by transporting the continuous sheet in a direction of transport,
the direction of transport being a direction in which the continuous sheet continues;
a processing device (45, 47) that processes a portion of the continuous sheet by the processing section at an interval in the direction of transport,
the portion being to be the absorbent article; and
a detecting device (48) that detects a trace of the processing left on the continuous sheet and that outputs a detection result,
the supplying section configured to adjust a direction-of-transport tension value (43, 43A, 43B, 44) of the continuous sheet based on the detection result in a way that if the detection result indicates that the trace of the processing is located downstream in the direction of transport with respect to a target position in the continuous sheet,
the tension value is increased, and
if the detection result indicates that the trace of the processing is located upstream in the direction of transport with respect to the target position,
the tension value is decreased,
the direction-of-transport tension value of the continuous sheet being a tension value at the time of transferring the continuous sheet to the processing section, wherein
the processing is defined as an upstream processing (45),
another processing is defined as a downstream processing (47),
the downstream processing is performed, at an interval in the direction of transport, at a position (P7) downstream in the direction of transport with respect to a position (P5) where the upstream processing is performed,
a portion of the continuous sheet that is to be the absorbent article is subject to the downstream processing, and
the trace of the processing is detected at a position (P48) downstream with respect to the position where the downstream processing is performed.

## Patentansprüche

1. Verfahren zur Herstellung eines blattförmigen Elements unter Verwendung eines kontinuierlichen Blatts (4a),
wobei das blattförmige Element einem saugfähigen Artikel (1) zugeordnet ist,
das Verfahren umfassend:
einen Zuführungsschritt (L4aS) zum Zuführen des kontinuierlichen Blatts,
wobei der Zuführungsschritt durch Übergeben des kontinuierlichen Blatts an einen Verarbeitungsabschnitt durch Transportieren des kontinuierlichen Blatts in einer Transportrichtung durchgeführt wird,
wobei die Transportrichtung eine Richtung ist, in der das kontinuierliche Blatt weiterläuft;
einen Verarbeitungsschritt (L4aK) zum Verarbeiten eines Abschnitts des kontinuierlichen Blatts durch den Verarbeitungsabschnitt in einem Intervall in der Transportrichtung,
wobei der Abschnitt der saugfähige Artikel sein soll,
wobei der Verarbeitungsschritt an einer vorbestimmten Position in der Transportrichtung durchgeführt wird;
einen Erfassungsschritt (48) zum Erfassen einer Spur der Verarbeitung, die auf dem kontinuierlichen Blatt verbleibt, und Ausgeben eines Erfassungsergebnisses,
wobei der Zuführungsschritt das Einstellen eines Transportrichtungsspannungswerts (43, 43A, 43B, 44) des kontinuierlichen Blatts auf Grundlage des Erfassungsergebnisses beinhaltet,
wobei der Transportrichtungsspannungswert des kontinuierlichen Blatts ein Spannungswert zu einem Zeitpunkt der Übergabe des kontinuierlichen Blatts an den Verarbeitungsabschnitt ist, wobei,
wenn das Erfassungsergebnis anzeigt, dass sich die Spur der Verarbeitung stromabwärts in der Transportrichtung in Bezug auf eine Zielposition in dem kontinuierlichen Blatt befindet,
der Spannungswert erhöht wird und
wenn das Erfassungsergebnis anzeigt, dass sich die Spur der Verarbeitung stromaufwärts in der Transportrichtung in Bezug auf die Zielposition befindet,
der Spannungswert verringert wird, wobei
die Verarbeitung als eine stromaufwärtige Verarbeitung (45) definiert ist,
eine andere Verarbeitung als eine stromabwärtige Verarbeitung (47) definiert ist,
die stromabwärtige Verarbeitung in einem Intervall in der Transportrichtung an einer Position (P7) stromabwärts in der Transportrichtung in Bezug auf eine Position (P5) durchgeführt wird, wo die stromaufwärtige Verarbeitung durchgeführt wird,
ein Abschnitt des kontinuierlichen Blatts, der der saugfähige Artikel sein soll, der stromabwärtigen Verarbeitung unterzogen wird, und
die Spur der Verarbeitung an einer Position (P48) stromabwärts in Bezug auf die Position, an der die stromabwärtige Verarbeitung durchgeführt wird, erfasst wird.

2. Verfahren zur Herstellung eines blattförmigen Elements, das einem saugfähigen Artikel zugeordnet ist, nach Anspruch 1, wobei
das kontinuierliche Blatt in dem Verarbeitungsabschnitt in einem vorbestimmten Transportpfad transportiert wird und
eine Transportrichtungslänge des Transportpfads konstant bleibt.

3. Verfahren zur Herstellung eines blattförmigen Elements, das einem saugfähigen Artikel zugeordnet ist, nach einem der Ansprüche 1 bis 2, wobei
in dem Zuführungsschritt eine Zuführvorrichtung, die das kontinuierliche Blatt von einer Materialspule zuführt, verwendet wird,
das kontinuierliche Blatt in der Zuführvorrichtung zugeführt und transportiert wird, während eine Zuführrichtung des kontinuierlichen Blatts die X-Richtung ist, wie von oben betrachtet,
das kontinuierliche Blatt in dem Verarbeitungsabschnitt transportiert wird, während die Transportrichtung des kontinuierlichen Blatts die Y-Richtung ist, wie von oben betrachtet, wobei die Y-Richtung die X-Richtung schneidet,
ein stabförmiges Element zum Ändern der Transportrichtung an einer Position in der Transportrichtung zwischen der Zuführvorrichtung und dem Verarbeitungsabschnitt angeordnet ist,
die Transportrichtung des kontinuierlichen Blatts durch Wickeln des kontinuierlichen Blatts um das Element zum Ändern der Transportrichtung von der X-Richtung zu der Y-Richtung wechselt und
die Spannungseinstellungsvorrichtung an einer Position in der Transportrichtung zwischen dem Element zum Ändern der Transportrichtung und dem Verarbeitungsabschnitt angeordnet ist.

4. Verfahren zur Herstellung eines blattförmigen Elements, das einem saugfähigen Artikel zugeordnet ist, nach Anspruch 3, wobei
die Spannungseinstellungsvorrichtung als eine erste Spannungseinstellungsvorrichtung definiert ist,
eine zweite Spannungseinstellungsvorrichtung zwischen der Zuführvorrichtung und dem Element zum Ändern der Transportrichtung in der Transportrichtung angeordnet ist und
die zweite Spannungseinstellungsvorrichtung zum Steuern eines Zuführbetriebs der Zuführvorrichtung dient, sodass ein Spannungswert des kontinuierlichen Blatts, das von der Zuführvorrichtung zugeführt wurde, ein vorbestimmter Wert wird.

5. Verfahren zur Herstellung eines blattförmigen Elements, das einem saugfähigen Artikel zugeordnet ist, nach einem der Ansprüche 1 bis 4, wobei in dem Verarbeitungsabschnitt eine Transportgeschwindigkeit, mit der das kontinuierliche Blatt in der Transportrichtung transportiert wird, geändert wird.

6. Verfahren zur Herstellung eines blattförmigen Elements, das einem saugfähigen Artikel zugeordnet ist, nach einem der Ansprüche 1 bis 5, wobei ein Zeitpunkt, zu dem die Verarbeitung in dem Verarbeitungsschritt durchgeführt wird, auf Grundlage des Erfassungsergebnisses verschoben wird.

7. Verfahren zur Herstellung eines blattförmigen Elements, das einem saugfähigen Artikel zugeordnet ist, nach einem der Ansprüche 1 bis 6, wobei
ein erstes kontinuierliches Blatt das kontinuierliche Blatt ist,
ein zweites kontinuierliches Blatt ein kontinuierliches Blatt ist, das sich von dem ersten kontinuierlichen Blatt unterscheidet, und
das zweite kontinuierliche Blatt an einer Zusammenführposition stromabwärts in der Transportrichtung in Bezug auf eine Position,
an der die Verarbeitung durchgeführt wird, übergeben wird und mit dem ersten kontinuierlichen Blatt verbunden wird.

8. Verfahren zur Herstellung eines blattförmigen Elements, das einem saugfähigen Artikel zugeordnet ist, nach Anspruch 7, wobei
das Verfahren ferner Folgendes umfasst:
einen zweiten Zuführungsschritt zum Zuführen des zweiten kontinuierlichen Blatts,
wobei der zweite Zuführungsschritt durch Übergeben des zweiten kontinuierlichen Blatts an einen zweiten Verarbeitungsabschnitt durch Transportieren des zweiten kontinuierlichen Blatts in einer Transportrichtung durchgeführt wird,
wobei die Transportrichtung eine Richtung ist, in der das zweite kontinuierliche Blatt weiterläuft;
einen zweiten Verarbeitungsschritt zum Durchführen einer zweiten Verarbeitung an einem Abschnitt des zweiten kontinuierlichen Blatts durch den zweiten Verarbeitungsabschnitt in einem Intervall in der Transportrichtung,
wobei der Abschnitt der saugfähige Artikel sein soll,
wobei der zweite Verarbeitungsschritt an einer vorbestimmten Position in der Transportrichtung durchgeführt wird; und
einen zweiten Erfassungsschritt zum Erfassen einer Spur der zweiten Verarbeitung, die auf dem zweiten kontinuierlichen Blatt verbleibt, und Ausgeben eines zweiten Erfassungsergebnisses,
wobei der zweite Zuführungsschritt das Einstellen eines Transportrichtungsspannungswert des kontinuierlichen Blatts auf Grundlage des zweiten Erfassungsergebnisses beinhaltet,
wobei der Transportrichtungsspannungswert des kontinuierlichen Blatts ein Spannungswert zum Zeitpunkt der Übergabe des kontinuierlichen Blatts an den zweiten Verarbeitungsabschnitt ist und
wobei sich die Zusammenführposition stromabwärts in der Transportrichtung in Bezug auf eine Position, an der die zweite Verarbeitung durchgeführt wird, befindet.

9. Verfahren zur Herstellung eines blattförmigen Elements, das einem saugfähigen Artikel zugeordnet ist, nach Anspruch 8, wobei
eine Spur der Verarbeitung, die auf dem ersten kontinuierlichen Blatt verbleibt, an einer Position stromabwärts in der Transportrichtung in Bezug auf die Zusammenführposition erfasst wird.

10. Vorrichtung zur Herstellung eines blattförmigen Elements unter Verwendung eines kontinuierlichen Blatts (4a),
wobei das blattförmige Element einem saugfähigen Artikel (1) zugeordnet ist,
die Vorrichtung umfassend:
einen Zuführungsabschnitt (L4aS), der das kontinuierliche Blatt durch Übergeben des kontinuierlichen Blatts an einen Verarbeitungsabschnitt (L4aK) durch Transportieren des kontinuierlichen Blatts in einer Transportrichtung zuführt,
wobei die Transportrichtung eine Richtung ist, in der das kontinuierliche Blatts weiterläuft;
eine Verarbeitungsvorrichtung (45, 47), die einen Abschnitt des kontinuierlichen Blatts durch den Verarbeitungsabschnitt in einem Intervall in der Transportrichtung verarbeitet,
wobei der Abschnitt der saugfähige Artikel sein soll; und
eine Erfassungsvorrichtung (48), die eine Spur der Verarbeitung, die auf dem kontinuierlichen Blatt verbleibt, erfasst und die ein Erfassungsergebnis ausgibt,
wobei der Zuführungsschritt dazu ausgelegt ist, einen Transportrichtungsspannungswerts (43, 43A, 43B, 44) des kontinuierlichen Blatts auf Grundlage des Erfassungsergebnisses auf eine Weise einzustellen, dass, wenn das Erfassungsergebnis anzeigt, dass sich die Spur der Verarbeitung stromabwärts in der Transportrichtung in Bezug auf eine Zielposition in dem kontinuierlichen Blatt befindet, der Spannungswert erhöht wird und
wenn das Erfassungsergebnis anzeigt, dass sich die Spur der Verarbeitung stromaufwärts in der Transportrichtung in Bezug auf die Zielposition befindet,
der Spannungswert verringert wird,
wobei der Transportrichtungsspannungswert des kontinuierlichen Blatts ein Spannungswert zum Zeitpunkt der Übergabe des kontinuierlichen Blatts an den Verarbeitungsabschnitt ist, wobei,
die Verarbeitung als eine stromaufwärtige Verarbeitung (45) definiert ist,
eine andere Verarbeitung als eine stromabwärtige Verarbeitung (47) definiert ist,
die stromabwärtige Verarbeitung in einem Intervall in der Transportrichtung an einer Position (P7) stromabwärts in der Transportrichtung in Bezug auf eine Position (P5) durchgeführt wird, wo die stromaufwärtige Verarbeitung durchgeführt wird,
ein Abschnitt des kontinuierlichen Blatts, der der saugfähige Artikel sein soll, der stromabwärtigen Verarbeitung unterzogen wird, und
die Spur der Verarbeitung an einer Position (P48) stromabwärts in Bezug auf die Position, an der die stromabwärtige Verarbeitung durchgeführt wird, erfasst wird.

## Revendications

1. Procédé de fabrication d'un élément de type feuille à l'aide d'une feuille continue (4a),
l'élément de type feuille étant associé à un article absorbant (1),
le procédé comprenant :
une étape de fourniture (L4aS) pour fournir la feuille continue,
l'étape de fourniture étant effectuée en transférant la feuille continue vers une section de traitement en transportant la feuille continue dans un sens de transport,
le sens de transport étant un sens dans lequel la feuille continue s'étend ;
une étape de traitement (L4aK) pour traiter une partie de la feuille continue par la section de traitement à un intervalle dans le sens de transport,
la partie devant être l'article absorbant,
l'étape de traitement étant effectuée à une position prédéterminée dans le sens de transport ;
une étape de détection (48) pour détecter une trace du traitement laissée sur la feuille continue et produire un résultat de détection,
l'étape de fourniture comportant le réglage d'une valeur de tension de sens de transport (43, 43A, 43B, 44) de la feuille de continue sur la base du résultat de détection,
la valeur de tension de sens de transport de la feuille continue étant une valeur de tension au moment du transfert de la feuille continue vers la section de traitement,
dans lequel
si le résultat de détection indique que la trace du traitement est située en aval dans le sens du transport par rapport à une position cible dans la feuille continue,
la valeur de tension est augmentée, et
si le résultat de détection indique que la trace du traitement est située en amont dans le sens de transport par rapport à la position cible,
la valeur de tension est diminuée, dans lequel
le traitement est défini comme un traitement en amont (45),
un autre traitement est défini comme un traitement en aval (47),
le traitement en aval est effectué, à un intervalle dans le sens de transport, à une position (P7) en aval dans le sens de transport par rapport à une position (P5) où le traitement en amont est effectué,
une partie de la feuille continue qui doit être l'article absorbant est soumise au traitement en aval, et
la trace du traitement est détectée à une position (P48) en aval par rapport à la position où le traitement en aval est effectué.

2. Procédé de fabrication d'un élément de type feuille associé à un article absorbant selon la revendication 1, dans lequel
dans la section de traitement, la feuille continue est transportée dans un trajet de transport prédéterminé, et
une longueur de sens de transport du trajet de transport reste constante.

3. Procédé de fabrication d'un élément de type feuille associé à un article absorbant selon l'une quelconque des revendications 1 et 2, dans lequel
lors de l'étape de fourniture, un dispositif d'alimentation qui alimente la feuille continue à partir d'une bobine de matériau est utilisé,
dans le dispositif d'alimentation, la feuille continue est alimentée et transportée tandis qu'un sens d'alimentation de la feuille continue est un sens X vu de dessus,
dans la section de traitement, la feuille continue est transportée tandis que le sens de transport de la feuille continue est un sens Y vu de dessus, le sens Y coupant le sens X,
un élément de changement de sens de transport en forme de barre est disposé à une position dans le sens de transport entre le dispositif d'alimentation et la section de traitement,
le sens de transport de la feuille continue change du sens X au sens Y en enroulant la feuille continue autour de l'élément de changement de sens de transport, et
le dispositif de réglage de la tension est disposé à une position dans le sens de transport entre l'élément de changement de sens de transport et la section de traitement.

4. Procédé de fabrication d'un élément de type feuille associé à un article absorbant selon la revendication 3, dans lequel
le dispositif de réglage de tension est défini comme un premier dispositif de réglage de tension,
un second dispositif de réglage de tension est disposé entre le dispositif d'alimentation et l'élément de changement de sens de transport dans le sens de transport, et
le second dispositif de réglage de tension est destiné à commander une opération d'alimentation du dispositif d'alimentation de sorte qu'une valeur de tension de la feuille continue qui a été alimentée par le dispositif d'alimentation devient une valeur prédéterminée.

5. Procédé de fabrication d'un élément de type feuille associé à un article absorbant selon l'une quelconque des revendications 1 à 4, dans lequel
dans la section de traitement, une vitesse de transport à laquelle la feuille continue est transportée dans le sens de transport est changée.

6. Procédé de fabrication d'un élément de type feuille associé à un article absorbant selon l'une quelconque des revendications 1 à 5, dans lequel
une synchronisation à laquelle le traitement est effectué au cours de l'étape de traitement est décalée sur la base du résultat de détection.

7. Procédé de fabrication d'un élément de type feuille associé à un article absorbant selon l'une quelconque des revendications 1 et 6, dans lequel
une première feuille continue est la feuille continue,
une seconde feuille continue est une feuille continue différente de la première feuille continue, et
à une position de fusion en aval dans le sens de transport par rapport à une position où le traitement est effectué,
la seconde feuille continue est transférée et collée avec la première feuille continue.

8. Procédé de fabrication d'un élément de type feuille associé à un article absorbant selon la revendication 7, dans lequel
le procédé comprend en outre :
une seconde étape de fourniture pour fournir la seconde feuille continue,
la seconde étape de fourniture étant effectuée en transférant la seconde feuille continue vers une seconde section de traitement en transportant la seconde feuille continue dans un sens de transport,
le sens de transport étant un sens dans lequel la seconde feuille continue s'étend ;
une seconde étape de traitement pour effectuer un second traitement sur une partie de la seconde feuille continue par la seconde section de traitement à un intervalle dans le sens de transport,
la partie devant être l'article absorbant,
la seconde étape de traitement étant effectuée à une position prédéterminée dans le sens de transport ; et
une seconde étape de détection pour détecter une trace du second traitement laissée sur la seconde feuille continue et produire un second résultat de détection,
la seconde étape de fourniture comporte le réglage d'une valeur de tension de sens de transport de la feuille continue sur la base du second résultat de détection,
la valeur de tension de sens de transport de la feuille continue étant une valeur de tension au moment du transfert de la feuille continue vers la seconde section de traitement, et
la position de fusion est située en aval dans le sens de transport par rapport à une position où le second traitement est effectué.

9. Procédé de fabrication d'un élément de type feuille associé à un article absorbant selon la revendication 8, dans lequel
une trace du traitement laissée sur la première feuille continue est détectée à une position en aval dans le sens de transport par rapport à la position de fusion.

10. Appareil de fabrication d'un élément de type feuille à l'aide d'une feuille continue (4a),
l'élément de type feuille étant associé à un article absorbant (1),
l'appareil comprenant :
une section de fourniture (L4aS) qui fournit la feuille continue en transférant la feuille continue vers une section de traitement (L4aK) en transportant la feuille continue dans un sens de transport,
le sens de transport étant un sens dans lequel la feuille continue s'étend ;
un dispositif de traitement (45, 47) qui traite une partie de la feuille continue par la section de traitement à un intervalle dans le sens de transport,
la partie devant être l'article absorbant ; et
un dispositif de détection (48) qui détecte une trace du traitement laissée sur la feuille continue et qui produit un résultat de détection,
la section d'alimentation configurée pour régler une valeur de tension de sens de transport (43, 43A, 43B, 44) de la feuille continue sur la base du résultat de détection d'une manière telle que si le résultat de détection indique que la trace du traitement est située en aval dans le sens de transport par rapport à une position cible dans la feuille continue,
la valeur de tension est augmentée, et
si le résultat de détection indique que la trace du traitement est située en amont dans le sens de transport par rapport à la position cible,
la valeur de tension est diminuée,
la valeur de tension de sens de transport de la feuille continue étant une valeur de tension au moment du transfert de la feuille continue vers la section de traitement,
dans lequel
le traitement est défini comme un traitement en amont (45),
un autre traitement est défini comme un traitement en aval (47),
le traitement en aval est effectué, à un intervalle dans le sens de transport, à une position (P7) en aval dans le sens de transport par rapport à une position (P5) où le traitement en amont est effectué,
une partie de la feuille continue qui doit être l'article absorbant est soumise au traitement en aval, et
la trace du traitement est détectée à une position (P48) en aval par rapport à la position où le traitement en aval est effectué.
